# EUROPEAN PATENT SPECIFICATION

(11) **EP 4 623 871 B1**
(45) Date of publication and mention of the grant of the patent: **09.12.2026**
(21) Application number: 25166850.5
(22) Date of filing: 27.03.2025
(51) Int. Cl.: A61F 2/44, A61F 2/46

(54) **INSERTION INSTRUMENTS**
EINSETZINSTRUMENTE
INSTRUMENTS D'INSERTION

(30) Priority: 28.03.2024 GB 202404565
(43) Date of publication of application: 01.10.2025
(73) Proprietor: Axis Spine Technologies Ltd, Leeds Yorkshire LS15 8ZB (GB)
(72) Inventor: HEUER, Frank, 70794 Filderstadt (DE)
(74) Representative: Peter, Kenneth William

(56) References cited:
- WO-A1-2021/014176
- WO-A1-2023/227877
- CN-A- 116 687 633

## Description

### Field of the Invention

The present invention relates to insertion instruments for inserting an intervertebral device into an intervertebral space and, more particularly but not exclusively, for inserting a modular intervertebral fusion device into an intervertebral space. The present disclosure relates to methods of inserting (not claimed) such an intervertebral fusion device into an intervertebral space by way of an insertion instrument.

### Background Art

Adjacent vertebrae in the spinal column are coupled to each other by a number of ligaments and the intervertebral disc. These anatomic structures hold the adjacent vertebrae together while allowing motion. Among these structures, the intervertebral disc functions as a cushion between the vertebrae whilst allowing for relative movement of the vertebrae. Problems with intervertebral discs arise from one or more of a range of diseases and conditions. A surgical procedure, such as spinal fusion, may be used to address such problems. The goals of spinal fusion include decompressing surrounding neural structures, re-establishing anatomic spinal alignment, and stabilising the motion segment by having one vertebral body fuse, or heal, to the adjacent vertebral body. A typical spinal fusion procedure involves partial or full removal of a problematic intervertebral disc and installation of an intervertebral device in the place of the partially or fully removed intervertebral disc in order to maintain the disc space height and alignment and facilitate the fusion of one vertebra to the next.

An intervertebral device, which is sometimes referred to as a cage, is inserted into the intervertebral space by approaching the spine from a particular direction depending on the procedure involved. By way of example, where the intervertebral device is an anterior lumbar interbody fusion (ALIF) device to be inserted during an ALIF procedure, an incision is made in one side of the abdomen and the abdominal muscles and the abdominal contents are then retracted to gain access to the spine. Regardless of the direction of approach to the spine from the incision according to the procedure involved, the disc is removed, the disc space is properly prepared and the intervertebral device is introduced into the patient's body by way of the incision before being inserted into the intervertebral space. Compared with other procedures, such as oblique lumbar interbody fusion and posterior lumbar interbody fusion procedures, the ALIF procedure allows a larger intervertebral device to be inserted into the intervertebral space. The typical ALIF device is therefore larger than other forms of intervertebral device.

Usually, the intervertebral device is held by an insertion instrument with the surgeon using the insertion instrument to introduce the intervertebral device into the patient's body, to guide the intervertebral device to the intervertebral space, and then to insert the intervertebral device into the intervertebral space. A known form of insertion instrument has arms which extend from the end of the insertion instrument held by the surgeon with the distal ends of the arms engaging respectively towards the superior and inferior ends of the intervertebral device. The arms of the insertion instrument are movable relative to each other. Where the intervertebral device is height and/or angle adjustable, the distal ends of the arms are moved by the surgeon to achieve a desired height and/or angle. Where intervertebral devices of fixed but respectively different height and/or angle are being used, the distal ends of the arms are moved by the surgeon to engage at different times with intervertebral devices of different height and/or angle.

Considering the procedure further and a modular intervertebral implant specifically, upon insertion of an intervertebral device in the intervertebral space with the insertion instrument, the intervertebral device is positioned in such a manner as to ensure the superior endplate component of the intervertebral device is aligned with and against the inferior endplate of the superior vertebra and the inferior endplate component of the intervertebral device is aligned with and against the superior endplate of the inferior vertebra. With the intervertebral endplate components in position, an appropriately sized core component is selected and inserted between the endplate components and such that the core component inter-engages with the superior and inferior endplate components. By providing the surgeon with a selection of core components of different heights and lordotic angles, the surgeon uses the assembled intervertebral device to achieve an anatomic correction of the position of the superior and inferior vertebrae relative to each other to restore the desired intervertebral height and open up the intervertebral foramen and thus decompress any compressed nerve roots. In cases where there has been anterior displacement of one vertebra relative to the other, posterior pedicle screw fixation can be employed. With screws implanted, the spine can be manipulated from the posterior side whereby the superior vertebra slides over the intervertebral device until the desired vertebral correction is achieved. When the desired vertebral correction is achieved, the intervertebral device is fixed to the superior vertebra using screws.

WO 2021/014173 discloses two forms of insertion instrument. In use of each form of insertion instrument, the core component is inserted into the intervertebral space and between the superior and inferior endplates by way of a core loader. The core loader with core component supported at its distal end is pushed between the upper and lower arms until the core component is properly located between the superior and inferior endplates. The present inventors have found in certain circumstances that although the superior and inferior endplates are held by the insertion instrument of WO 2021/014173 in their proper position in the intervertebral space before insertion of the core component, insertion of the core component may cause one, other or both of the superior and inferior endplates to move from their proper position. For example, the superior and inferior endplates may become misaligned with each other. Further examples of insertion instruments for intervertebral fusion devices are disclosed in CN 116 687 633 A, WO 2021/014176 A1 and WO 2023/227877 A1.

The present invention has been devised in light of the inventors' appreciation of the above-mentioned problem. It is therefore an object for the present invention to provide an improved insertion instrument for inserting an intervertebral fusion device into an intervertebral space. It is a further object for the present invention to provide an insertion instrument for inserting a modular intervertebral fusion device into an intervertebral space, the modular intervertebral fusion device comprising a superior endplate, an inferior endplate and a core component which, in use, is received between the superior and inferior endplates. It is a yet further object for the present disclosure to provide a method (not claimed) of inserting an intervertebral fusion device, and more specifically, a modular intervertebral fusion device into an intervertebral space by way of an insertion instrument. While the implantation methods described herein do not form part of the invention, they are disclosed as they represent useful background for understanding the invention.

### Statement of Invention

The present invention relates to a device as claimed hereafter. Preferred embodiments of the invention are set forth in the dependent claims. According to a first aspect of the present invention there is provided an insertion instrument for inserting a modular intervertebral fusion device or components thereof into an intervertebral space, the insertion instrument comprising:
a superior support and an inferior support, the superior and inferior supports opposing each other;
a superior arm mounted on the superior support;
an inferior arm mounted on the inferior support, the superior and inferior arms extending in generally a same direction from the superior and inferior supports whereby the superior and inferior arms oppose each other, a distal end of each of the superior and inferior arms configured to engage at a respective location on a modular intervertebral fusion device or with a respective component of a modular intervertebral fusion device; and
a mechanical linkage arrangement mechanically coupled to the superior and inferior supports and extending therebetween, the mechanical linkage arrangement comprising first to fourth linkage arms, wherein
each of the superior and inferior arms comprises an arm hinge between its distal end and the respective one of the superior and inferior supports, the arm hinge rotating about an axis which is substantially orthogonal to the longitudinal axis of the respective arm and to a direction of separation of the superior and inferior arms,
distal ends of the first and second linkage arms are rotatably coupled to the superior support at spaced apart locations thereon, the distal end of at least one of the first and second linkage arms coupled to the superior support for relative translation of superior support and respective distal end to thereby change a separation of the distal ends of the first and second linkage arms,
distal ends of the third and fourth linkage arms are rotatably coupled to the inferior support at spaced apart locations thereon, the distal end of at least one of the third and fourth linkage arms coupled to the inferior support for relative translation of inferior support and respective distal end to thereby change a separation of the distal ends of the third and fourth linkage arms, and
the first to fourth linkage arms are mechanically coupled to one another each at a location on the respective linkage arm spaced apart from its distal end whereby a separation is changeable between a) and b), where a) is the distal ends of the first and second linkage arms and b) is the distal ends of the third and fourth linkage arms.

The insertion instrument is for inserting a modular intervertebral fusion device, such as an ALIF device, or components thereof into an intervertebral space. The insertion instrument comprises a superior support, an inferior support, a superior arm and an inferior arm. The superior and inferior supports oppose each other. The superior arm is mounted on a superior support and the inferior arm is mounted on the inferior support. More specifically, the superior arm may be mounted at its proximal end on the superior support and the inferior arm may be mounted at its proximal end on the inferior support. Furthermore, each of the superior and inferior arms may be mounted such that the proximal end of each arm is substantially immovable relative to its respective superior or inferior support.

The insertion instrument also comprises a mechanical linkage arrangement which is mechanically coupled to the superior and inferior supports and extends between the superior and inferior supports. The mechanical linkage arrangement comprises first to fourth linkage arms. As described further below, the mechanical linkage arrangement is coupled to the superior and inferior supports by way of the first to fourth linkage arms to provide relative movement of the superior and inferior supports. The superior and inferior arms extend in generally a same direction from the superior and inferior supports whereby the superior and inferior arms oppose each other. A distal end of each of the superior and inferior arms is configured to engage at a respective location on a modular intervertebral fusion device or with a respective component of a modular intervertebral fusion device. The insertion instrument is thus configured to support a modular intervertebral fusion device or components thereof, such as a superior endplate on the distal end of the superior arm and an inferior endplate on the distal end of the inferior arm. In use, the insertion instrument is held by clinician and the thus supported modular intervertebral fusion device is or components thereof are inserted into an intervertebral space.

If a modular intervertebral fusion device has a large angle, a distal portion of the superior arm is at a correspondingly large angle to a distal portion of the inferior arm. Where the superior and inferior arms are long, as is typically required of an insertion instrument for an ALIF device, the separation between the superior and inferior arms at their proximal ends, i.e. at the ends opposite the distal ends supporting intervertebral fusion device, may be considerable. Each of the superior and inferior arms therefore comprises an arm hinge between its distal end and the respective one of the superior and inferior supports. The arm hinge in each of the superior and inferior arms allows there to be a significant angle between the superior and inferior arms at the intervertebral device whilst allowing for reduction in an extent to which there is separation between the superior and inferior arms at their proximal ends. Reduction in extent to which there is separation between the superior and inferior arms at their proximal ends may allow for the surgical incision to be smaller and hence less traumatic to the patient. Where the insertion instrument is being used in the like of an L5-S1 procedure, the presence of an arm hinge in the inferior arm reduces likelihood of the inferior arm colliding with the pubic symphysis.

The arm hinge rotates about an axis which is substantially orthogonal to the longitudinal axis of the respective arm and to a direction of separation of the superior and inferior arms. The axis of the arm hinge thus extends in the transverse direction. Having an arm hinge on each of the superior and inferior arms may allow for rotation of the distal ends of the superior and inferior arms about their respective arm hinges, such as during insertion of a core component of the intervertebral fusion device between superior and inferior endplates held by the insertion instrument, whereby likelihood of misalignment of the held superior and inferior endplates may be reduced. Aside from this advantage, having an arm hinge on each of the superior and inferior arms instead of an arm hinge on only one of the superior and inferior arms may result in less separation of the superior and inferior arms during insertion of the intervertebral fusion device. Less separation of superior and inferior arms during insertion of the intervertebral fusion device may mean a smaller surgical wound opening and/or narrower path to the intervertebral space.

The axis of the arm hinge of the superior arm may be substantially parallel to the axis of the arm hinge of the inferior arm. Furthermore, the axis of the arm hinge of the superior arm may be spaced apart from the superior support by substantially a same distance as the axis of the arm hinge of the inferior arm is spaced apart from the inferior support. The axes of the two arm hinges may therefore be substantially in registration. Having thus configured arm hinges may provide for symmetry of movement of the distal ends of the superior and inferior arms about their respective arm hinges, such as during insertion of a core component of the intervertebral fusion device between superior and inferior endplates held by the insertion instrument. The symmetry of movement may be about a plane which is parallel to the longitudinal axes of the superior and inferior arms and is substantially orthogonal to a direction of separation of the superior and inferior arms.

Distal ends of the first and second linkage arms are rotatably coupled to the superior support at spaced apart locations on the superior support. As further described below, the distal ends of the first and second linkage arms may be rotatably coupled to the superior support at locations on the superior support which are spaced apart in the direction of the longitudinal axis of the superior arm. Alternatively and as further described below, the distal ends of the first and second linkage arms may be rotatably coupled to the superior support at locations on the superior support which are spaced apart in a direction parallel to the axis of the arm hinge. The distal end of at least one of the first and second linkage arms is coupled to the superior support for relative translation of superior support and respective distal end to thereby change a separation of the distal ends of the first and second linkage arms.

Distal ends of the third and fourth linkage arms are coupled to the inferior support in a corresponding fashion to coupling of the distal ends of the first and second linkage arms to the superior support. Therefore, the distal ends of the third and fourth linkage arms are rotatably coupled to the inferior support at spaced apart locations on the inferior support. The distal ends of the third and fourth linkage arms may be rotatably coupled to the inferior support at spaced apart locations on the inferior support in a fashion corresponding to the first and second linkage arms as described above. The distal end of at least one of the third and fourth linkage arms is coupled to the inferior support for relative translation of inferior support and respective distal end to thereby change a separation of the distal ends of the third and fourth linkage arms.

The mechanical linkage arrangement is further configured such that the first to fourth linkage arms are mechanically coupled to one another each at a location on the respective linkage arm spaced apart from its distal end and whereby a separation is changeable between a) and b), where a) is the distal ends of the first and second linkage arms and b) is the distal ends of the third and fourth linkage arms. The insertion instrument is thus configured by way of mechanical coupling of distal ends of the first to fourth linkage arms to their respective support and by way of mechanical coupling of the first to fourth linkage arms to one another each at a location spaced apart from its distal end for movement together and apart of the distal ends of the first and second linkage arms relative to the distal ends of the third and fourth linkage arms. Consequently, the superior and inferior supports move together and apart to allow for relative positioning of the superior and inferior endplates while the two arm hinges reduce likelihood of misalignment of the superior and inferior endplates.

Furthermore, the mechanical linkage arrangement may be configured such that there is substantially no relative movement of superior and inferior supports in a direction parallel to the axis of at least one of the arm hinges. Alternatively or in addition, the mechanical linkage arrangement may be configured such that there is substantially no relative movement of superior and inferior supports in a direction of the longitudinal axis of at least one of the superior and inferior arms. The insertion instrument may therefore allow relative linear translation of the superior and inferior supports substantially in only one direction, i.e. together and apart. Likelihood of misalignment of the held superior and inferior endplates may thus be reduced.

The insertion instrument may comprise a further mechanical linkage arrangement which is mechanically coupled to the superior and inferior supports and extends therebetween. The insertion instrument may thus comprise first and second mechanical linkage arrangements which are spaced apart from each other. As further described below, the first and second mechanical linkage arrangements may be spaced apart in a direction parallel to the axis of at least one of the arm hinges. Alternatively and as further described below, the first and second mechanical linkage arrangements may be spaced apart in a longitudinal direction of at least one of the superior and inferior arms. Spacing apart of the first and second mechanical linkage arrangements may either define a space for receiving a tool, such as a core loader, between the superior or inferior arms and/or provide structure for supporting such a tool. Having such a tool receiving space or tool supporting structure between the superior and inferior supports may provide for more balanced distribution of forces on the superior and inferior supports and arms as a core component is inserted than in insertion instruments in which a tool, such as a core loader, is attached to one of the superior and inferior supports. Furthermore, such a tool receiving space or tool supporting structure may result in a core component being presented at a better trajectory to the superior and inferior endplates.

As described above, the first to fourth linkage arms are mechanically coupled to one another each at a location on the respective linkage arm spaced apart from its distal end whereby a separation is changeable between a) and b), where a) is the distal ends of the first and second linkage arms and b) is the distal ends of the third and fourth linkage arms. More specifically, the first and second linkage arms may be coupled to each other for their relative rotation, and the third and fourth linkage arms may be coupled to each other for their relative rotation. The first and second linkage arms may be coupled to each other for relative rotation in opposite directions. The third and fourth linkage arms may be coupled to each other for relative rotation in opposite directions. Furthermore, the first and second linkage arms may be coupled to each other for their relative rotation at their proximal ends, and the third and fourth linkage arms may be coupled to each other for their relative rotation at their proximal ends.

In a first embodiment, the first and second linkage arms may be coupled to each other for their relative rotation about a first linkage arm axis, and the third and fourth linkage arms may be coupled to each other for their relative rotation about a second linkage arm axis. The first and second linkage arm axes may substantially coincide. Furthermore, the first and second linkage arm axes may be substantially parallel to a longitudinal axis of at least one of the superior and inferior arms.

In addition, the first and second linkage arm axes may be substantially centrally located between the superior and inferior supports. Locating the first and second linkage arm axes centrally between the superior and inferior supports may contribute to balancing of forces between the superior and inferior supports and superior and inferior arms during insertion of a core component between superior and inferior endplates.

The distal ends of the first and third linkage arms may be towards one transverse side of the superior and inferior supports and the distal ends of the second and fourth linkage arms may be towards the other transverse side of the superior and inferior supports.

In the first embodiment the first and fourth linkage arms may be immovably attached to each other whereby the first and fourth linkage arms rotate together relative to the second and third linkage arms. The second and third linkage arms may be immovably attached to each other whereby the second and third linkage arms rotate together relative to the first and fourth linkage arms. Such a configuration may substantially prevent relative rotation of the superior and inferior supports about an axis substantially coinciding with at least one of the first and second linkage arm axes. The first and fourth linkage arms may be of substantially the same length. The first and fourth linkage arms may be integrally formed with each other. The second and third linkage arms may be integrally formed with each other.

Each of the first and fourth linkage arms may be immovably attached at their proximal ends to a first axle and, more specifically, may be immovably attached to the first axle such that the first and fourth linkage arms extend from opposite sides of the first axle. Each of the second and third linkage arms may be immovably attached at their proximal ends to a second axle and, more specifically, may be immovably attached to the second axle such that the second and third linkage arms extend from opposite sides of the second axle.

The first and second axles may be disposed in a direction substantially parallel to the longitudinal axis of at least one of the superior and inferior arms. The first and second axles may be supported such that they are relatively rotatable and about the same axis. Furthermore, each of the first and second axles may define a substantially centrally located axle bore which extends substantially parallel to the longitudinal axis of at least one of the superior and inferior arms. In use, the axle bores allow for passage therethrough of a tool, such as a core loader. Having axle bores which are centrally located between the superior and inferior supports may contribute to balancing of forces between the superior and inferior supports and superior and inferior arms during insertion of a core component between superior and inferior endplates.

In the first embodiment, the distal end of each of the first to fourth linkage arms is coupled to the respective one of the superior and inferior supports for rotation relative to the respective support and for relative translation of respective support and respective distal end. More specifically, relative translation of respective support and respective distal end may be in a direction substantially orthogonal to the longitudinal axis of at least one of the superior and inferior arms and to the direction of separation of the superior and inferior arms. Each of the superior and inferior supports may define two spaced apart channels and the distal end of each of the first to fourth linkage arms may define a protrusion, each protrusion received in a respective one of the channels for rotation therein and for relative translation. The direction of the channel may determine the direction of relative translation of respective support and respective distal end.

The present inventors have found a configuration of insertion instrument which substantially prevents relative rotation of the superior and inferior supports about an axis which is substantially orthogonal to the longitudinal axes of the superior and inferior arms and to a direction of separation of the superior and inferior arms may in certain circumstances provide insufficient degrees of freedom of relative movement of superior and inferior arms to provide for ease of proper relative disposition of superior and inferior endplates in an intervertebral space. A second embodiment of insertion instrument has been developed in light of this appreciation.

According to the second embodiment, the mechanical linkage arrangement may be configured to allow relative rotation of the superior and inferior supports about an axis which is substantially orthogonal to the longitudinal axes of the superior and inferior arms and to a direction of separation of the superior and inferior arms. Allowing for relative rotation of the superior and inferior supports about an axis which is substantially orthogonal to the longitudinal axes of the superior and inferior arms and to a direction of separation of the superior and inferior arms has been found to be inessential but advantageous in certain circumstances. For example, such relative rotation of the superior and inferior supports may reduce an extent to which the distal ends of the superior and inferior arms are rotated about their respective arm hinges. This configuration may be more forgiving when force is applied during core component insertion because the core component is more readily steered between the superior and inferior arms.

In the second embodiment, the first to fourth linkage arms may be mechanically coupled to one another for rotation relative to one another, each of the first to fourth linkage arms so coupled at a location on the respective linkage arm spaced apart from its distal end. Furthermore, the mechanical linkage arrangement may be configured for rotation of the first and third linkage arms independently of the second and fourth linkage arms, the first and third linkage arms rotating together albeit relative to each other and the second and fourth linkage arms rotating together albeit relative to each other.

The distal ends of the first and third linkage arms may be closer to the superior and inferior arms than the distal ends of the second and fourth linkage arms.

Each of the first to fourth linkage arms may be mechanically coupled for rotation about a respective one of first to fourth linkage arm axes each of which is substantially orthogonal to the longitudinal axis of at least one of the superior and inferior arms and to a direction of separation of the superior and inferior arms. The first to fourth linkage arm axes may be substantially parallel and spaced apart from one another. More specifically, the first to fourth linkage arm axes may extend through a linkage arm plane parallel to the longitudinal axes of the superior and inferior arms and a direction of separation of the superior and inferior arms. Furthermore, the first to fourth linkage arm axes may define in the linkage arm plane four corners of a rectangle.

In view of the first to fourth linkage arms being mechanically coupled to one another for rotation relative to one another and the first and third linkage arms rotating independently of the second and fourth linkage arms, the distal end of only one of the first and second linkage arms may be coupled to the superior support for relative translation of superior support and the distal end. Furthermore, the distal end of only one of the third and fourth linkage arms may be coupled to the inferior support for relative translation of inferior support and the distal end. This configuration may allow for movement of the superior and inferior supports together and apart and for their relative rotation about an axis which is substantially orthogonal to the longitudinal axis of at least one of the superior and inferior arms and to a direction of separation of the superior and inferior arms but with substantially no relative linear translation of superior and inferior supports in the direction of the longitudinal axis of at least one of the superior and inferior arms.

The distal end of the second linkage arm which is further from the superior arm than the distal end of the first linkage arm may be coupled to the superior support for relative translation of the superior support and the distal end. The superior support may define a superior channel and the distal end of the second linkage arm may define a protrusion which is slidably received in the superior channel. The superior channel may extend substantially parallel to the longitudinal axis of the superior arm.

Furthermore, the distal end of the first linkage arm may be coupled to the superior support for relative rotation of the superior support and the distal end and for substantially no relative translation of the superior support and the distal end. The superior support may define a superior support bore and the distal end of the first linkage arm may define a protrusion which is snugly received in the superior support bore for rotation within the superior support bore.

The distal end of the fourth linkage arm which is further from the inferior arm than the distal end of the third linkage arm may be coupled to the inferior support for relative translation of inferior support and the distal end. The inferior support may define an inferior channel and the distal end of the fourth linkage arm may define a protrusion which is slidably received in the inferior channel. The inferior channel may extend substantially parallel to the longitudinal axis of the inferior arm.

Furthermore, the distal end of the third linkage arm may be coupled to the inferior support for relative rotation of the inferior support and the distal end and for substantially no relative translation of the inferior support and the distal end. The inferior support may define an inferior support bore and the distal end of the third linkage arm may define a protrusion which is snugly received in the inferior support bore for rotation within the inferior support bore.

As described above, the mechanical linkage arrangement may be configured for rotation of the first and third linkage arms together albeit relative to each other, and for rotation of the second and fourth linkage arms together albeit relative to each other. The first and third linkage arms may therefore be mechanically coupled for their rotation together and the second and fourth linkage arms may therefore be mechanically coupled for their rotation together. More specifically, the first and third linkage arms may be mechanically coupled for rotation in opposite directions. Furthermore, the second and fourth linkage arms may be mechanically coupled for rotation in opposite directions. Configuration of the mechanical linkage arrangement in this fashion may provide for substantial symmetry of movement of the superior and inferior supports relative to each other, such as during insertion of a core component of the intervertebral fusion device between superior and inferior endplates held by the insertion instrument. The substantial symmetry of movement may be about a plane which extends between the superior and inferior supports and in the transverse direction, and which is equally spaced from the superior and inferior supports. The substantial symmetry of movement of the superior and inferior supports relative to each other may correspond to balancing of forces exerted during insertion of the like of the core component between the superior and inferior supports. Furthermore, and where there is relative rotation of the superior and inferior supports, there may be substantial symmetry of relative rotation of the superior and inferior supports whereby there is balancing of forces exerted during insertion of the like of the core component between the superior and inferior supports. Substantial symmetry of relative rotation of the superior and inferior supports may be particularly advantageous where the intervertebral fusion device when assembled defines an angle of 20 degrees or more.

Each of the proximal ends of the first to fourth linkage arms may define a gearwheel or part thereof, teeth of the gearwheel of the first linkage arm engaging with teeth of the gearwheel of the third linkage arm, and teeth of the gearwheel of the second linkage arm engaging with teeth of the gearwheel of the fourth linkage arm. Rotation of the first linkage arm may thus cause rotation of the third linkage arm and vice-versa, and rotation of the second linkage arm may thus cause rotation of the fourth linkage arm and vice-versa.

The superior and inferior supports and the mechanical linkage arrangement may removably attach to the superior and inferior arms. The superior and inferior arms may be used with the superior and inferior supports and the mechanical linkage arrangement removed, for example, to align held superior and inferior endplates in the intervertebral space, such as with an alignment tool which is inserted between the superior and inferior arms. The superior and inferior supports and the mechanical linkage arrangement may then be removably attached to the superior and inferior arms for core component trialling followed by core component insertion.

One of the superior and inferior arms may be longer than the other of the superior and inferior arms whereby the longer arm extends beyond an end of the respective one of the superior and inferior supports opposite the end of the respective support from which the respective arm extends, and the shorter one of the superior and inferior arms extends no further that an end of the respective one of the superior and inferior supports opposite the end of the respective support from which the respective arm extends. The part of the longer one of the superior and inferior arms that extends beyond the respective one of the superior and inferior supports may be configured to support the core component, such as before the core component is attached to a trial core insertion tool or a core loader. The part of the longer one of the superior and inferior arms that extends beyond the respective one of the superior and inferior supports may define structure which inter-engages with the core component. The superior arm may be longer than the inferior arm.

The arm hinge may be free to rotate about its axis whereby the arm hinge lacks the like of a lock for holding a distal portion of the respective arm at a set angle to a proximal portion of the respective arm.

The arm hinge may be located closer to the distal end of the respective arm than to the respective one of the superior and inferior supports. More specifically, the arm hinge may be located no further than 30 percent along the length of the respective arm from the distal end of the arm. A distance of between 15 and 20 percent from the distal end of the arm has been found to be advantageous in certain circumstances and more specifically to provide for a typical patient effective reduction in risk of collision with the pubic symphysis.

The superior arm may be configured to grip a superior endplate component between oppositely directed sides of the superior endplate component. Alternatively or in addition, the inferior arm may be configured to grip an inferior endplate component between oppositely directed sides of the inferior endplate component.

Oppositely directed sides of the endplate component may be gripped by a gripping arrangement. The gripping arrangement may comprise first and second fingers which extend from a distal end of a respective arm, the first and second fingers spaced apart to sufficient extent to receive an endplate component therebetween.

The first and second fingers may be movably mounted on the respective arm whereby a separation of the first and second fingers is changed. An endplate component may be readily received between the first and second fingers when the first and second fingers are more widely spaced. The endplate component may be gripped by the first and second fingers when the first and second fingers are less widely spaced.

A finger may be rotatably mounted on the respective arm, an axis of rotation of the finger extending in a direction of separation of the superior and inferior arms. The finger may be rotatably mounted towards its proximal end. A separation of distal ends of the first and second fingers may change upon rotation of the first and second fingers.

A distal end of a finger and a side of the endplate component may be configured to form a detent which temporarily and substantially prevents the endplate component when gripped from moving in the longitudinal axis of the respective arm. One of the distal end of the finger and the side of the endplate component may define a protrusion and the other of the distal end of the finger and the side of the endplate component may define a recess. The protrusion or recess may be defined on a side of the finger which faces the other one of the first and second fingers. The finger on the respective arm may move between a first position in which the protrusion is not received in the recess and a second position in which the protrusion is received in the recess. The finger may define the protrusion and the side of the endplate component may define the recess.

The gripping arrangement may further comprise a user operable compression mechanism which moves the finger between the first and second positions. Where the finger is rotatably mounted on the respective arm, the compression mechanism may rotate the finger between the first and second positions. Where each of the first and second fingers is rotatably mounted on the respective arm, the compression mechanism may rotate each of the first and second fingers between the first and second positions. The distal ends of the first and second fingers may be more widely spaced apart in the first position and the distal ends of the first and second fingers may be less widely spaced apart in the second position.

The compression mechanism may comprise a compression body which is movably attached to its respective arm, the compression body mounted on the arm for movement along the arm. Movement of the compression body along the arm may move at least one finger between the first and second positions.

When the compression body is moved away from the respective one of the superior and inferior supports, the compression body may bear against at least one of the first and second fingers to move the finger from the first position to the second position. The finger may define a first finger shoulder against which the compression body bears to move the finger. A distal end of the compression body may bear against the first finger shoulder.

When the compression body is moved towards the respective one of the superior and inferior supports, the compression body may bear against at least one of the first and second fingers to move the finger from the second position to the first position. The finger may define a second finger shoulder against which the compression body bears to move the finger. The compression body may define a compression body shoulder which bears against the second finger shoulder. Having such a user operable means of moving the finger from the locked second position to the unlocked first position may be more effective at overcoming resistance presented by surrounding tissue than, for example, relying on spring bias. Furthermore, having such a user operable means of moving the first and second fingers from the locked second position to the unlocked first position may provide for greater likelihood of substantially simultaneous movement of the first and second fingers from the first position to the second position than, for example, relying on a spring bias for each of the first and second fingers.

The arm may comprise a compression mechanism driving arrangement which is supported on its respective arm for movement relative to the arm. The compression mechanism driving arrangement may comprise a first driving member and a second driving member which are hingedly coupled to each other. The first and second driving members may rotate relative to each other about a compression axis. The compression axis may substantially coincide with the axis of the arm hinge of the respective arm.

The first driving member may be hingedly coupled at its distal end to a proximal end of the second driving member. The first driving member may be coupled at its proximal end to a user operable actuator, such as a lever.

The second driving member may be attached to the compression mechanism whereby the compression mechanism moves with the second driving member. Where the compression mechanism comprises a compression body, the second driving member may be attached to the compression body and, more specifically, may be integrally formed with the compression body. Movement of the first driving member may cause the second driving member to move back and forth along the respective arm whereby the compression body moves at least one of the first and second fingers between the first and second positions.

When the first and second fingers are in the second position, the movable nature of the first and second fingers may make it difficult to align the first and second fingers with the oppositely directed sides of the endplate component. One of the distal end of the respective arm and the anterior or posterior side of the endplate component may define an alignment protrusion and the other of the distal end of the respective arm and the anterior or posterior side of the endplate component may define an alignment recess. The alignment protrusion and the alignment recess may be shaped for reception of the alignment protrusion in the alignment recess on movement of the endplate component towards the distal end of the respective arm. Engagement of the alignment protrusion and the alignment recess may align the endplate component with the distal end of the respective arm and before the first and second fingers are moved from their first position to their second position. The distal end of the respective arm may define two alignment protrusions which are spaced apart in the transverse direction and the anterior or posterior side of the endplate component may define two alignment recesses which are spaced apart in the transverse direction.

As described above, the insertion instrument is for inserting an intervertebral device or components thereof. Therefore, and according to a second aspect of the present invention, there is provided an intervertebral device insertion assembly comprising an insertion instrument according to the first aspect and an intervertebral device or components thereof, the intervertebral device configured for attachment of each of the superior and inferior arms at respective spaced apart locations on the intervertebral device or on components thereof. The superior arm and a superior endplate of the intervertebral device may be configured for gripping of the superior endplate by the superior arm as described above. Furthermore, the inferior arm and an inferior endplate of the intervertebral device may be configured for gripping of the inferior endplate by the inferior arm as described above.

The intervertebral device may be a trial core component. The intervertebral device may be an implantable core component. The intervertebral device insertion assembly may comprise plural core components, such as plural trial core components and plural implantable core component. Core components of different heights and of different lordotic angles may be required to attend to different surgical requirements.

The intervertebral device insertion assembly may comprise an elongate trial core insertion tool which is configured to hold a trial core component and to be moved in the direction of the longitudinal axis of at least one of the superior and inferior arms when received between the superior and inferior arms.

The intervertebral device insertion assembly may comprise an elongate core loader tool which is configured to hold a core component, and to be moved in the direction of the longitudinal axis of at least one of the superior and inferior arms when received between the superior and inferior arms.

Further embodiments of the second aspect of the present invention may comprise one or more features of the first aspect of the present invention.

According to a third aspect of the present disclosure there is provided a method (not claimed) of inserting a modular intervertebral fusion device into an intervertebral space by way of an insertion instrument, the insertion instrument comprising a superior arm mounted on a superior support, an inferior arm mounted on an inferior support, the superior and inferior supports opposing each other whereby the superior and inferior arms extend in generally a same direction from the superior and inferior supports and the superior and inferior arms oppose each other, and a mechanical linkage arrangement mechanically coupled to the superior and inferior supports and extending therebetween, the mechanical linkage arrangement comprising first to fourth linkage arms, the method comprising:
engaging the distal end of each of the superior and inferior arms at a respective location on a modular intervertebral fusion device or with a respective component of a modular intervertebral fusion device to thereby support the intervertebral fusion device or components thereof;
moving the superior and inferior supports together or apart to provide relative movement of the superior and inferior arms; and
introducing the modular intervertebral fusion device or components thereof into the intervertebral space by way of the insertion instrument, wherein
each of the superior and inferior arms comprises an arm hinge between its distal end and the respective one of the superior and inferior supports, the arm hinge rotating about an axis which is substantially orthogonal to the longitudinal axis of the respective arm and to a direction of separation of the superior and inferior arms,
distal ends of the first and second linkage arms are rotatably coupled to the superior support at spaced apart locations thereon, the distal end of at least one of the first and second linkage arms coupled to the superior support for relative translation of superior support and respective distal end to thereby change a separation of the distal ends of the first and second linkage arms,
distal ends of the third and fourth linkage arms are rotatably coupled to the inferior support at spaced apart locations thereon, the distal end of at least one of the third and fourth linkage arms coupled to the inferior support for relative translation of inferior support and respective distal end to thereby change a separation of the distal ends of the third and fourth linkage arms, and
the first to fourth linkage arms are mechanically coupled to one another each at a location on the respective linkage arm spaced apart from its distal end whereby a separation is changeable between a) and b), where a) is the distal ends of the first and second linkage arms and b) is the distal ends of the third and fourth linkage arms.

The method of inserting a modular intervertebral fusion device into an intervertebral space by way of an insertion instrument comprises engaging the distal end of each of superior and inferior arms of the insertion instrument at a respective location on a modular intervertebral fusion device, such as an ALIF device, or with a respective component of a modular intervertebral fusion device to thereby support the intervertebral fusion device or components thereof. The method also comprises moving superior and inferior supports of the insertion instrument together or apart to provide relative movement of the superior and inferior arms. The method further comprises introducing the modular intervertebral fusion device or components thereof into the intervertebral space by way of the insertion instrument. The method may yet further comprise inserting a component, such as a core component, between intervertebral fusion device components, such as superior and inferior endplates, held in the intervertebral space by the insertion instrument. A tool, such as a core loader, may be used to insert the core component between the fusion device components. Insertion of the core component is likely to apply forces to the superior and inferior arms and perhaps also to the superior and inferior supports. The presence of an arm hinge in each of the superior and inferior arms and the configuration of the mechanical linkage arrangement and its mechanical coupling to the superior and inferior supports may provide for balancing of the applied forces between the superior and inferior arms. Balancing of the applied forces reduces likelihood of the intervertebral fusion device components supported by the superior and inferior arms becoming misplaced. For example, there may be reduced likelihood of misalignment of superior and inferior endplates.

Further embodiments of the third aspect of the present disclosure may comprise one or more features of the first aspect of the present invention.

### Brief Description of Drawings

Further features and advantages of the present invention will become apparent from the following specific description, which is given by way of example only and with reference to the accompanying drawings, in which:
Figure 1 is a perspective view of a first embodiment of insertion instrument;
Figure 2A is a view from above of an arm of the first embodiment of Figure 1 and when the endplate component gripper is open;
Figure 2B is a view from below of the arm of the first embodiment of Figure 1 and when the endplate component gripper is open;
Figure 2C is a view from above of the arm of the first embodiment of Figure 1 and when the endplate component gripper is closed;
Figure 3A is a detailed view of the distal end of the arm of the first embodiment of Figure 1 and with an endplate component in situ;
Figure 3B is the detailed view of Figure 3A and when the endplate component is gripped by the endplate component gripper;
Figure 4A is a perspective view of the superior and inferior supports and the mechanical linkage arrangements of the first embodiment when the mechanical linkage arrangements are open;
Figure 4B is a side view of the arrangement shown in Figure 4A;
Figure 4C is an end view of the arrangement shown in Figure 4A;
Figure 4D is a perspective view of the arrangement of Figure 4A when the mechanical linkage arrangements are closed;
Figure 5A is a perspective view of the first embodiment of insertion instrument and held components of an intervertebral fusion device at a first stage of use;
Figure 5B is a perspective view of the first embodiment of insertion instrument and held components of the intervertebral fusion device at a second stage of use;
Figure 5C is a perspective view of the first embodiment of insertion instrument and held components of the intervertebral fusion device at a third stage of use;
Figure 5D is a perspective view of the first embodiment of insertion instrument and held components of the intervertebral fusion device at a fourth stage of use;
Figure 5E is a perspective view of the intervertebral fusion device when the first embodiment of insertion instrument has been removed at a fifth stage of use;
Figure 6 is a flow chart representation of a surgical procedure involving use of the first embodiment of insertion instrument;
Figure 7 is a perspective view of a second embodiment of insertion instrument;
Figure 8A is a view from above of the superior arm of the second embodiment of Figure 7 and when the endplate component gripper is closed;
Figure 8B is a view from below of the superior arm of the second embodiment of Figure 7 and when the endplate component gripper is closed;
Figure 9A is a view from above of the inferior arm of the second embodiment of Figure 7 and when gripping an inferior component;
Figure 9B is a view from below of the inferior arm of the second embodiment of Figure 7 and when gripping an inferior component;
Figure 10A is a perspective view of the superior and inferior supports and the mechanical linkage arrangements of the second embodiment when the mechanical linkage arrangements are open;
Figure 10B is a side view of the arrangement shown in Figure 10A;
Figure 10C is an end view of the arrangement shown in Figure 10A;
Figure 10D is perspective view of the superior and inferior supports and the mechanical linkage arrangements of the second embodiment when the mechanical linkage arrangements are open and angled;
Figure 10E is a perspective view of the arrangement of Figure 10A when the mechanical linkage arrangements are closed;
Figure 10F is a side view of the linkage arms of one of the mechanical linkage arrangements of the second embodiment;
Figure 11A is a perspective view of the second embodiment of insertion instrument and held components of an intervertebral fusion device at a first stage of use;
Figure 11B is a perspective view of the second embodiment of insertion instrument and held components of the intervertebral fusion device at a second stage of use;
Figure 11C is a perspective view of the second embodiment of insertion instrument and held components of the intervertebral fusion device at a third stage of use;
Figure 11D is a perspective view of the second embodiment of insertion instrument and held components of the intervertebral fusion device at a fourth stage of use;
Figure 11E is a perspective view of the second embodiment of insertion instrument and held components of the intervertebral fusion device at a fifth stage of use; and
Figure 12 is a flow chart representation of a surgical procedure involving use of the second embodiment of insertion instrument.

### Description of Embodiments

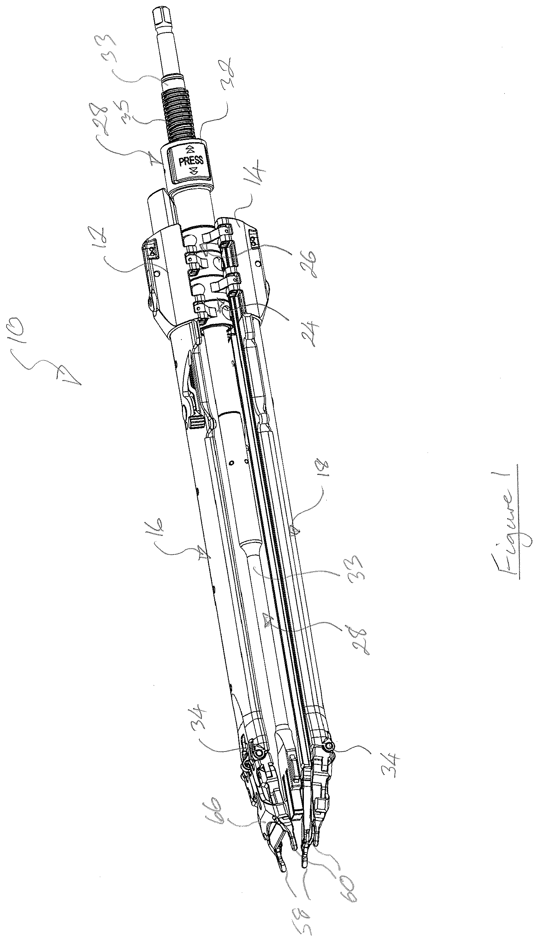

A first embodiment 10 of insertion instrument and parts thereof are shown in Figures 1 to 5E. Referring first to Figure 1, the first embodiment 10 of insertion instrument comprises a superior support 12 and an inferior support 14 which oppose each other. A superior arm 16 is removably mounted on the superior support 12 and an inferior arm 18 is removably mounted on the inferior support 14. Each of the superior and inferior arms has a length of 320 mm. The form and function of the superior arm 16 are the same as the form and function of the inferior arm 18 except as now described. The superior arm 16 defines a keyway at each of its two transverse sides and each of the transverse sides of a core component 30 is shaped to be slidably received in a respective one of the keyways whereby the core component is held by the superior arm and the core component slides along the superior arm during insertion of the core component. The superior and inferior arms 16, 18 extend in generally the same direction from the superior and inferior supports 12, 14 whereby the superior and inferior arms oppose each other. As described further below, the distal end of each of the superior and inferior arms 16, 18 is configured to grip a respective one of a superior endplate component 20 and an inferior endplate component 22 of a modular anterior lumbar interbody fusion (ALIF) device. Although the description of the present embodiment involves insertion of an ALIF device, any other intervertebral fusion device, such as an oblique lumbar interbody fusion device or a posterior lumbar interbody fusion device, may be inserted. The first embodiment 10 of insertion instrument also comprises first and second mechanical linkage arrangements 24, 26 each of which is mechanically coupled to and extends between the superior and inferior supports 12, 14. The first and second mechanical linkage arrangements 24, 26 are disposed in a direction substantially parallel to longitudinal axes of the superior and inferior arms 16, 18.

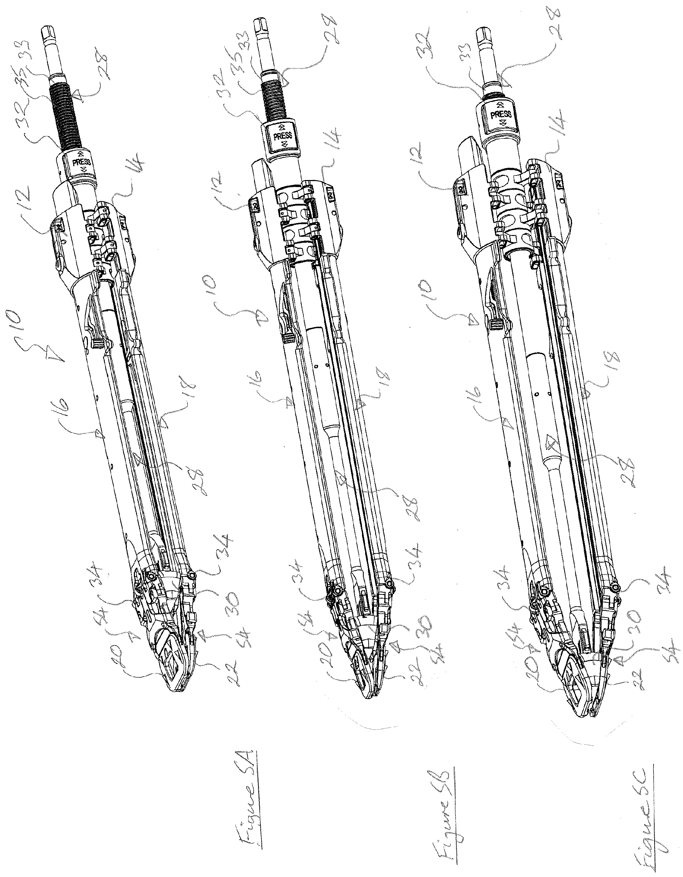

The first embodiment 10 of insertion instrument further comprises a core loader 28 which holds the core component 30 of the modular ALIF device at its distal end (see Figures 5A to 5D) and is supported by the first and second mechanical linkage arrangements 24, 26 such that the core loader extends centrally between the superior and inferior supports 12, 14 and centrally between the superior and inferior arms 16, 18. The core loader 28 comprises a handle 32 at its proximal end. In use, and as described further below, the clinician grips the handle 32 and rotates the proximal end of the core loader 28 such that the held core component 30 is advanced along the superior and inferior arms 16, 18 and then between the superior and inferior endplate components 20, 22. The handle 32 is removably attached to the superior and inferior supports 12, 14 such that it does not rotate relative to the superior and inferior supports 12, 14. The core loader 28 is structured such that the handle 32 defines a threaded through bore and the core loader 28 comprises an elongate core loader member 33 which is received through the threaded through bore of the handle 32. The elongate core loader member 33 defines a threaded portion 35 on its outside surface and towards its proximal end. The threaded portion 35 threadedly engages with the threaded through bore of the handle 32. Rotation of the elongate core loader member 33 relative to the handle 32 in a first direction advances the core component 30 holding distal end of the core loader 28 towards the distal ends of the superior and inferior arms 16, 18. Rotation of the elongate core loader member 33 relative to the handle 32 in a second opposite direction withdraws the core component 30 holding distal end of the core loader 28 from the distal ends of the superior and inferior arms 16, 18.

Each of the superior and inferior arms 16, 18 comprises an arm hinge 34 about 20 percent of the distance along the respective arm from the distal end of the arm. Each arm hinge 34 rotates about a respective arm hinge axis which is substantially orthogonal to the longitudinal axis of the respective arm 16, 18 and to a direction of separation of the superior and inferior arms 16, 18. The two arm hinges 34 are parallel to each other. Furthermore, the arm hinge 34 of the superior arm 16 is spaced apart from the distal end of the superior arm by substantially the same distance as the arm hinge 34 of the inferior arm 18 is spaced apart from the distal end of the inferior arm. The axes of the two arm hinges 34 are therefore substantially in registration.

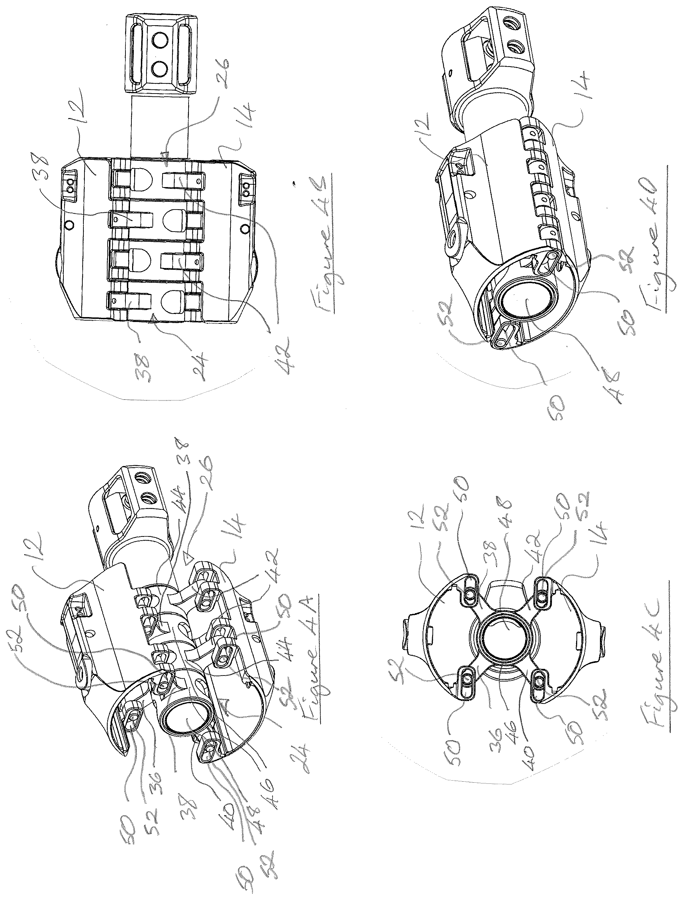

Referring in particular to Figures 4A to 4C, each of the first and second mechanical linkage arrangements 24, 26 comprises a first linkage arm 36, a second linkage arm 38, a third linkage arm 40 and a fourth linkage arm 42. As described further below, distal ends of the first and second linkage arms 36, 38 are rotatably coupled to the superior support 12 at spaced apart locations on the superior support and distal ends of the third and fourth linkage arms 40, 42 are rotatably coupled to the inferior support 14 at spaced apart locations on the inferior support. Furthermore, the rotatable coupling of the distal ends of the first and second linkage arms 36, 38 to the superior support 12 also allows for relative translation of the superior support and the distal ends whereby separation of the distal ends of the first and second linkage arms changes. Likewise, the rotatable coupling of the distal ends of the third and fourth linkage arms 40, 42 to the inferior support 14 allows for relative translation of the inferior support and the distal ends whereby separation of the distal ends of the third and fourth linkage arms changes. The first to fourth linkage arms 36, 38, 40, 42 of each of the first and second mechanical linkage arrangements 24, 26 are mechanically coupled to one another each at its proximal end whereby a separation is changeable between a) and b), where a) is the distal ends of the first and second linkage arms 36, 38 and b) is the distal ends of the third and fourth linkage arms 40, 42. The change in separation between a) and b) can be appreciated by comparing Figure 4A, which shows the first and second mechanical linkage arrangements 24, 26 when open, with Figure 4D, which shows the first and second mechanical linkage arrangements when closed.

Referring again in particular to Figures 4A to 4C, the first and fourth linkage arms 36, 42 are immovably attached at their proximal ends to a first axle 44 such that the first and fourth linkage arms extend from opposite sides of the first axle. The second and third linkage arms 38, 40 are immovably attached at their proximal ends to a second axle 46 such that the second and third linkage arms extend from opposite sides of the second axle. The first and second axles 44, 46 are disposed relative to each other such that the first and third linkage arms 36, 40 extend on one transverse side of the superior and inferior supports 12, 14 and the second and fourth linkage arms 38, 42 extend on the opposite transverse side of the superior and inferior supports. The first and fourth linkage arms 36, 42 and the first axle 44 are of the same form and size as the second and third linkage arms 38, 40 and the second axle 46. The first and second axles 44, 46 are rotatably attached to each other such that they are relatively rotatable about the same linkage arm axis which is substantially parallel to the longitudinal axes of the superior and inferior arms 16, 18. The first to fourth linkage arms 36, 38, 40, 42 are thus coupled to one another at their proximal ends such that the first and fourth linkage arms 36, 42 rotate together, the second and third linkage arms 38, 40 rotate together and the first and fourth linkage arms rotate relative to the second and third linkage arms. As can be seen from Figures 4A, 4C and 4D, each of the first and second axles 44, 46 of the first and second mechanical linkage arrangements 24, 26 defines a central through axle bore 48 with the four through axle bores in registration with one another. As can be seen from Figures 1 and 5A to 5C, the four through axle bores define a passage which receives the core loader 28.

Referring again in particular to Figures 4A, 4C and 4D, each of the superior support 12 and the inferior support 14 defines two spaced apart channels 50 for each of the first and second mechanical linkage arrangements 24, 26. The channels 50 on each of the superior support 12 and the inferior support 14 are spaced apart from each other towards transverse sides of the respective support 12, 14. Each channel 50 extends in the transverse direction, i.e. in a direction substantially orthogonal to the longitudinal axis of the respective one of the superior and inferior arms 16, 18 and to the direction of separation of the superior and inferior arms. The distal end of each of the first to fourth linkage arms 36, 38, 40, 42 defines a protrusion 52 which extends from the respective distal end in the direction of the longitudinal axis of the respective one of the superior and inferior arms 16, 18. As the superior and inferior supports 12, 14 move together and apart, each protrusion 52 rotates in its respective channel and moves along its respective channel.

When the superior and inferior arms 16, 18 or the superior and inferior supports 12, 14 are pushed apart or pushed together, as described further below with reference to Figures 5A to 5C, the first and second axles 44, 46 of the first and second mechanical linkage arrangements 24, 26 rotate about the linkage arm axis to constrain movement of the superior and inferior supports relative to each other. The first and second mechanical linkage arrangements 24, 26 allow for relative translation of the superior and inferior supports 12, 14 together and apart but allow substantially no relative translation of the superior and inferior supports in the direction of the longitudinal axes of the superior and inferior arms 16, 18 and substantially no relative rotation of the superior and inferior supports about an axis which extends in the transverse direction, i.e. an axis which is substantially orthogonal to the longitudinal axis of the superior and inferior arms 16, 18 and to the direction of separation of the superior and inferior arms.

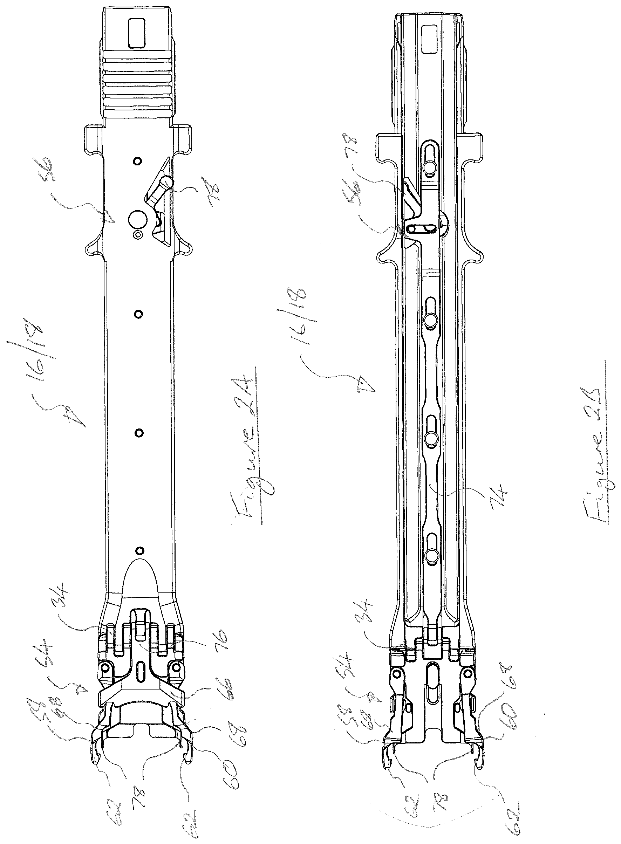

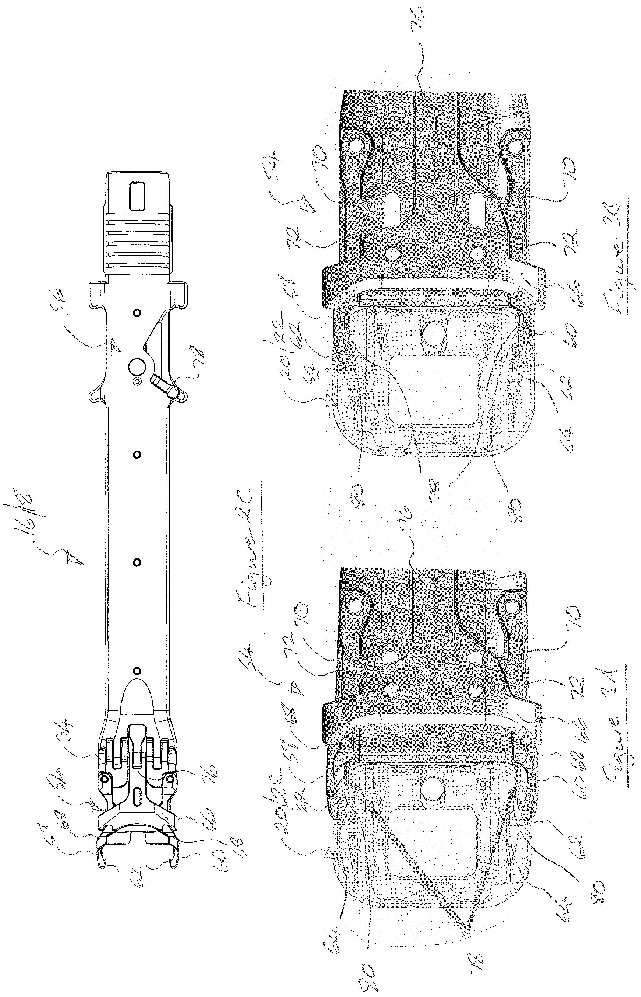

Figures 2A to 2C are views of one of the superior arm 16 and the inferior arm 18 of the first embodiment 10 of insertion instrument. Figure 2A is a view of the arm 16, 18 from above and when an endplate component gripper is open. Figure 2B is a view of the arm 16, 18 from below and when the endplate component gripper is open. Figure 2C is a view of the arm 16, 18 from above and when the endplate component gripper is closed.

As described above, the arm 16, 18 comprises an arm hinge 34 about 20 percent of the distance along the respective arm from the distal end of the arm and which joins a distal portion 54 of the arm to a proximal portion 56 of the arm. The arm hinge 34 rotates about a respective arm hinge axis which is substantially orthogonal to the longitudinal axis of the arm 16, 18 and to a direction of separation of the superior and inferior arms 16, 18. The distal portion 54 of the arm 16, 18 therefore rotates relative to the proximal portion 56 of the arm. In view of the superior and inferior arms 16, 18 opposing each other, as shown in Figures 1 and 5A to 5D, rotation of the distal portion 54 of both superior and inferior arms 16, 18 relative to their proximal portions 56 changes an angle between the two distal portions 54 whereby an angle between the superior and inferior endplate components 20, 22 is changed. A lordotic angle for the modular ALIF device can thus be set.

As mentioned above, the distal end of each of the superior and inferior arms 16, 18 is configured to grip a respective one of a superior endplate component 20 and an inferior endplate component 22 of a modular anterior lumbar interbody fusion (ALIF) device. Referring to Figures 3A and 3B, each of the superior and inferior arms 16, 18 is configured to grip a respective one of the superior and inferior endplate components 20, 22 between oppositely directed sides of the respective endplate component. Oppositely directed sides of the respective endplate component 20, 22 are gripped by a gripping arrangement which comprises first and second fingers 58, 60 which extend from a distal end of the distal portion 54 of the respective arm. The first and second fingers 58, 60 are located at transverse sides of the distal portion 54 whereby the first and second fingers are spaced apart to sufficient extent to receive the endplate component 20, 22 therebetween. Each of the first and second fingers 58, 60 is rotatably mounted at its proximal end on the distal portion 54 of the respective arm for rotation about an axis which extends in a direction of separation of the superior and inferior arms. The separation in the transverse direction of distal ends of the first and second fingers 58, 60 changes upon rotation of the first and second fingers. Figures 2A, 2B and 3A show the first and second fingers 58, 60 when more widely separated, i.e. when the endplate component gripper is open, and Figures 2C and 3B show the first and second fingers 58, 60 when less widely separated, i.e. when the endplate component gripper is closed.

Referring to Figures 3A and 3B, the distal end of each finger 58, 60 defines a protrusion 62 and each of the transverse sides of the respective endplate component 20, 22 defines a recess 64 which is shaped to receive a respective finger 58, 60. The protrusions 62 protrude towards each other from opposing faces of their respective fingers 58, 60. When the first and second fingers 58, 60 are rotated to their open condition, the respective endplate component 20, 22 is received between the first and second fingers. When the first and second fingers 58, 60 are rotated from their open condition to their closed condition, each protrusion 62 is received in the respective recess 64 whereby the respective endplate component 20, 22 is gripped and its removal from the distal portion 54 of the respective arm 16, 18 is resisted.

Each of the superior and inferior arms 16, 18 further comprises a user operable compression mechanism which is operative to rotate the first and second fingers 58, 60 between their open and closed conditions. The compression mechanism comprises a compression body 66 which is movably attached to the distal portion 54 of the respective arm 16, 18 for movement along the distal portion away from and towards the respective one of the superior and inferior supports 12, 14. Each of the first and second fingers 58, 60 defines a first finger shoulder 68 towards its distal end. When the compression body 66 is moved away from the respective one of the superior and inferior supports 12, 14, a distal end of the compression body bears against first finger shoulders 68 of the first and second fingers 58, 60 to rotate the first and second fingers from their open condition to their closed condition. Each of the first and second fingers 58, 60 also defines a second finger shoulder 70 towards its proximal end. The compression body 66 defines a two transversely spaced apart compression body shoulders 72 each of which is oriented towards the respective one of the superior and inferior supports 12, 14 and in a transverse direction. When the compression body 66 is moved towards the respective one of the superior and inferior supports 12, 14, each compression body shoulder 72 bears against a respective second finger shoulder 70 to rotate the first and second fingers 58, 60 from their closed condition to their open condition.

Referring now to Figures 2A to 2C, each of the superior and inferior arms 16, 18 further comprises a compression mechanism driving arrangement which is supported on its respective arm for movement relative to the arm. The compression mechanism driving arrangement comprises a first driving member 74 and a second driving member 76 which are hingedly coupled to each other. The first and second driving members 74, 76 rotate relative to each other about a compression axis which substantially coincides with the axis of the arm hinge 34 of the respective arm 16, 18. The first driving member 74 is hingedly coupled at its distal end to a proximal end of the second driving member 76. The first driving member 74 is mounted for movement along the respective arm 16, 18 and is coupled at its proximal end to a user operable lever 78. The second driving member 76 is integrally formed with the compression body 66 whereby the compression body extends away from the proximal end of the second driving member. Movement of the lever 78 in a first direction moves the first driving member 74 along the respective arm 16, 18 away from the respective one of the superior and inferior supports 12, 14 which causes movement of the second driving member 76 and the compression body 66 away from the respective one of the superior and inferior supports 12, 14. As described above, movement of the compression body 66 away from the respective one of the superior and inferior supports 12, 14 moves the first and second fingers 58, 60 from their open condition to their closed condition. Movement of the lever 78 in a second opposite direction moves the first driving member 74 along the respective arm 16, 18 towards the respective one of the superior and inferior supports 12, 14 which causes movement of the second driving member 76 and the compression body 66 towards the respective one of the superior and inferior supports 12, 14. As described above, movement of the compression body 66 towards the respective one of the superior and inferior supports 12, 14 moves the first and second fingers 58, 60 from their closed condition to their open condition. Rotation of the second driving member 76 relative to the first driving member 74 about the compression axis allows the second driving member to rotate with the distal portion 54 of the respective arm.

The distal end of the distal portion 54 of the respective arm defines two alignment protrusions 78 which protrude in the longitudinal direction of the arm and are located towards transverse sides of the distal portion whereby the alignment protrusions 78 are spaced apart from each other in the transverse direction. The anterior side of the respective endplate component 20, 22 defines two alignment recesses 80 which are spaced apart in the transverse direction and to the same extent as the two alignment protrusions 78 are spaced apart. Each alignment protrusion 78 is a snug fit in a respective alignment recess 80. When the respective endplate component 20, 22 is brought into engagement with the distal portion 54 of the respective arm 16, 18, the alignment protrusions 78 are received in their respective alignment recesses 80 to thereby align the endplate component 20, 22 with the distal portion 54 and before the first and second fingers 58, 60 are moved from their open condition to their closed condition.

Parts of the insertion instrument 10 are formed from medical grade stainless steel. In other forms of the insertion instrument 10 the superior and inferior arms 16, 18 are formed from a polymeric material, for example PEEK, PAEK, PEAK, PEKK, PE, PU, PPSU, PA, ABS or the like, with or without fibrous material, such as glass, carbon or nylon) to reinforce the polymeric matrix. Forming the superior and inferior arms 16, 18 from a polymeric material improves visibility under x-ray inspection. Further to this, parts of the insertion instrument 10 are coated with diamond like carbon (DLC) to reduce light reflection and thereby improve inspection by way of optical instruments. For the same reason, parts of the insertion instrument 10 are roughened by sandblasting to reduce direct light reflection.

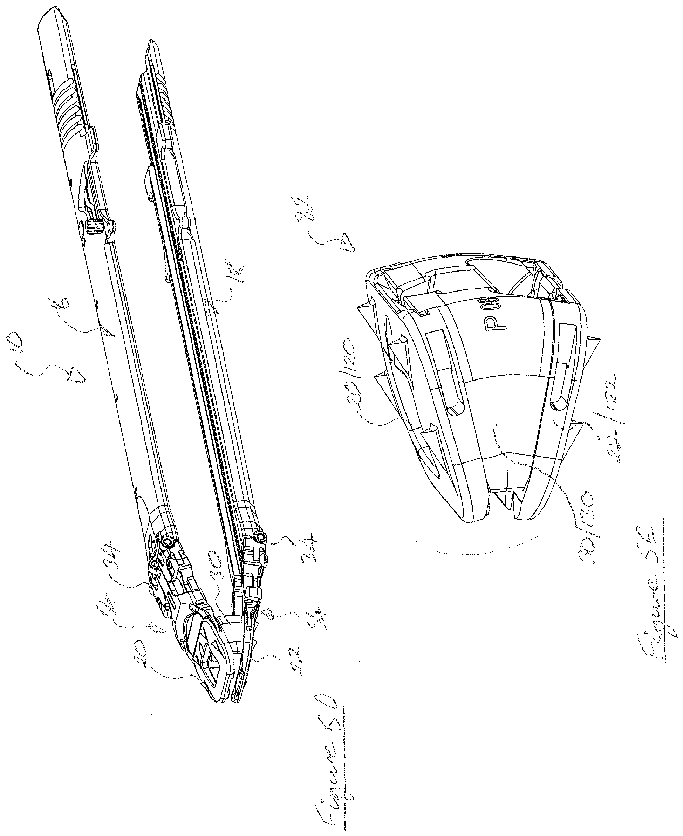

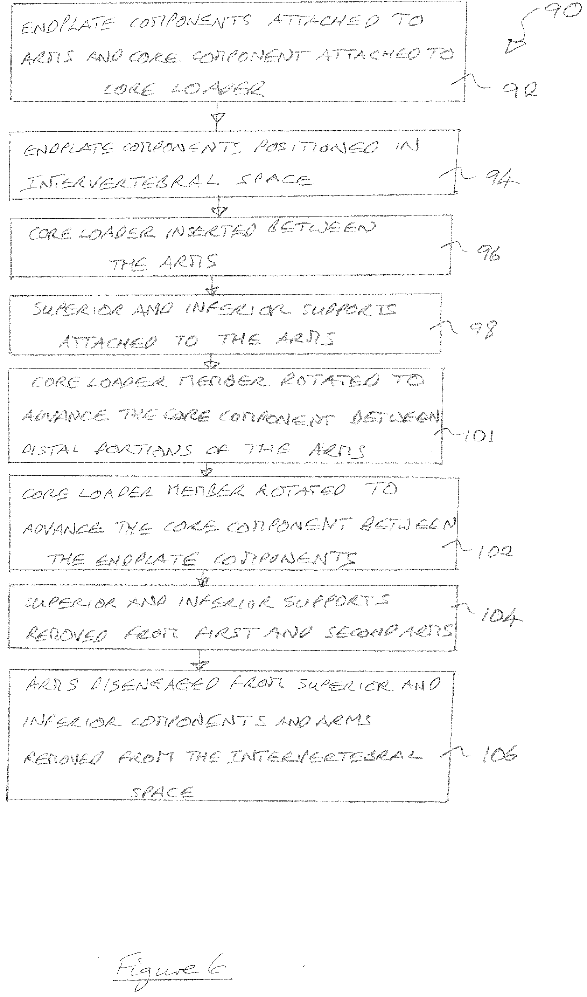

A method of use (not claimed) of the first embodiment 10 of insertion instrument in an ALIF procedure will now be described with reference to Figures 5A to 5E and the flow chart 90 of Figure 6. A superior endplate component 20 is attached to the distal end of the superior arm 16 and an inferior endplate component 22 is attached to the distal end of the inferior arm 18, as described above, 92. A core component 30 is attached to the distal end of the core loader 28, 92. The superior and inferior arms 16, 18 are inserted into a surgical aperture and the held superior and inferior endplate components 20, 22 are positioned in an intervertebral space 94. The core loader 28 is then inserted between the superior and inferior arms 16, 18 until the held core component 30 is just beyond the arm hinges 34, 96. The superior and inferior supports 12, 14 and the first and second mechanical linkage arrangements 24, 26 are then attached to the superior and inferior arms 16, 18, 98 whereby the insertion instrument 10 is in the condition shown in Figure 5A. The elongate core loader member 33 is rotated by the clinician relative to the handle 32 of the core loader 28 to advance the held core component 30 between the distal portions 54 of the superior and inferior arms 101 as shown in Figure 5B. The superior and inferior arms 16, 18 have now been pushed apart to some extent and the distal portions 54 and their held superior and inferior endplate components 20, 22 have now been rotated relative to each other to some extent. The elongate core loader member 33 is rotated further by the clinician to advance the held core component 30 between the held superior and inferior endplate components 20, 22, 102 as shown in Figure 5C. The superior and inferior arms 16, 18 have now been pushed apart to further extent and the distal portions 54 and their held superior and inferior endplate components 20, 22 have now been further rotated to receive the core component 30 between them. The clinician then removes the core loader 28, and the superior and inferior supports 12, 14 and the first and second mechanical linkage arrangements 24, 26 from the superior and inferior arms 16, 18, 104 whereby the insertion instrument 10 is in the condition shown in Figure 5D. Finally, the clinician disengages the distal ends of the superior and inferior arms 16, 18 from the superior and inferior endplate components 20, 22, as described above, and removes the superior and inferior arms from the intervertebral space 106. The assembled ALIF device 82, which is shown in Figure 5E, is thus installed in the intervertebral space.

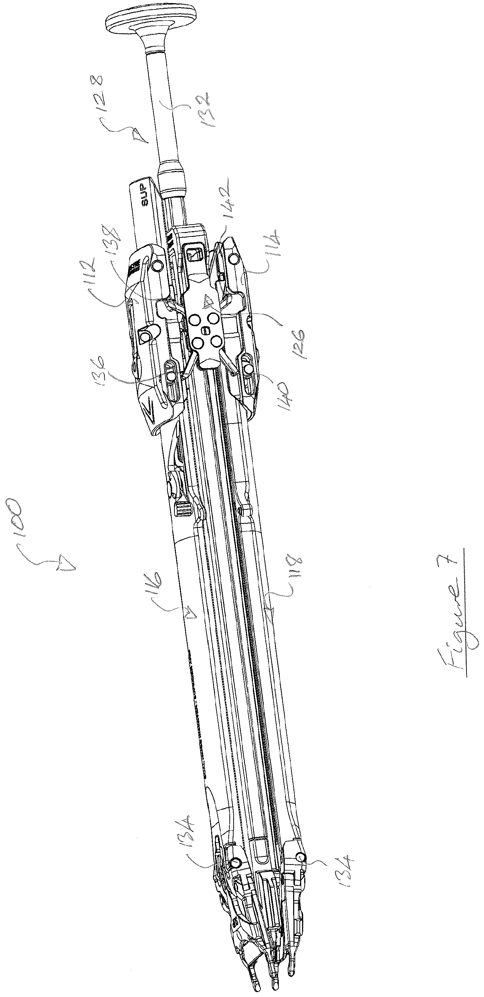

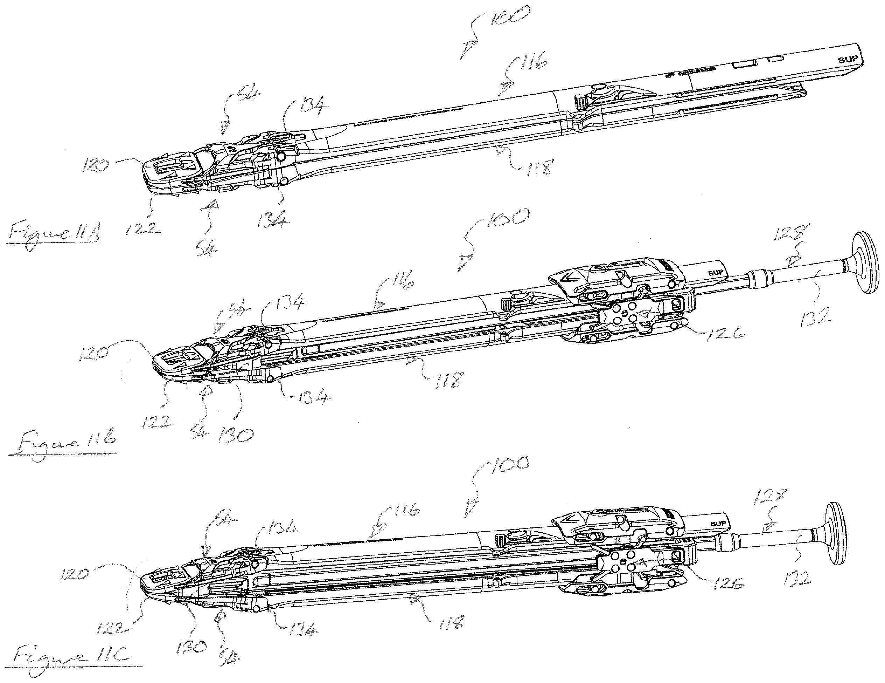

A second embodiment 100 of insertion instrument and parts thereof are shown in Figures 7 to 11E. Referring first to Figure 7, the second embodiment 100 of insertion instrument comprises a superior support 112 and an inferior support 114 which oppose each other. A superior arm 116 is removably mounted on the superior support 112 and an inferior arm 118 is removably mounted on the inferior support 114. The form and function of the superior arm 116 are the same as the form and function of the inferior arm 118 except in respect of greater length of the superior component at its proximal end and as can be seen, in particular, from Figure 11A.

The superior arm has a length of 350 mm and the inferior arm has a length of 320 mm. The superior and inferior arms 116, 118 extend in generally the same direction from the superior and inferior supports 112, 114 whereby the superior and inferior arms oppose each other. As described further below, the distal end of each of the superior and inferior arms 116, 118 is configured to grip a respective one of a superior endplate component 120 and an inferior endplate component 122 of a modular anterior lumbar interbody fusion (ALIF) device. Although the description of the present embodiment involves insertion of an ALIF device, any other intervertebral fusion device, such as an oblique lumbar interbody fusion device or a posterior lumbar interbody fusion device, may be inserted. The second embodiment 100 of insertion instrument also comprises first and second mechanical linkage arrangements 124, 126 each of which is mechanically coupled to and extends between the superior and inferior supports 112, 114. The first and second mechanical linkage arrangements 124, 26 extend between the superior and inferior supports 112, 114 at opposite transverse sides of the superior and inferior supports whereby the first and second mechanical linkage arrangements are spaced apart from each other in the transverse direction.

The second embodiment 100 of insertion instrument further comprises a core loader 128 which holds a core component 130 of the modular ALIF device at its distal end (see Figures 11A to 11D) and is supported by the first and second mechanical linkage arrangements 124, 126 such that the core loader extends centrally between the superior and inferior supports 112, 114 and centrally between the superior and inferior arms 116, 118. The core loader 128 comprises a handle 132 at its proximal end. In use, and as described further below, the clinician grips the handle 132 and pushes the core loader 28 such that the held core component 130 is advanced between the superior and inferior arms 116, 118 and then between the superior and inferior endplate components 120, 122. Alternatively or in addition, the end of the handle 132 is tapped by the clinician to advance the held core component 130.

Each of the superior and inferior arms 116, 118 comprises an arm hinge 134 about 20 percent of the distance along the respective arm from the distal end of the arm. Each arm hinge 134 rotates about a respective arm hinge axis which is substantially orthogonal to the longitudinal axis of the respective arm 116, 118 and to a direction of separation of the superior and inferior arms 116, 118. The two arm hinges 134 are parallel to each other. Furthermore, the arm hinge 134 of the superior arm 116 is spaced apart from the distal end of the superior arm by substantially the same distance as the arm hinge 134 of the inferior arm 118 is spaced apart from the distal end of the inferior arm. The axes of the two arm hinges 134 are therefore substantially in registration.

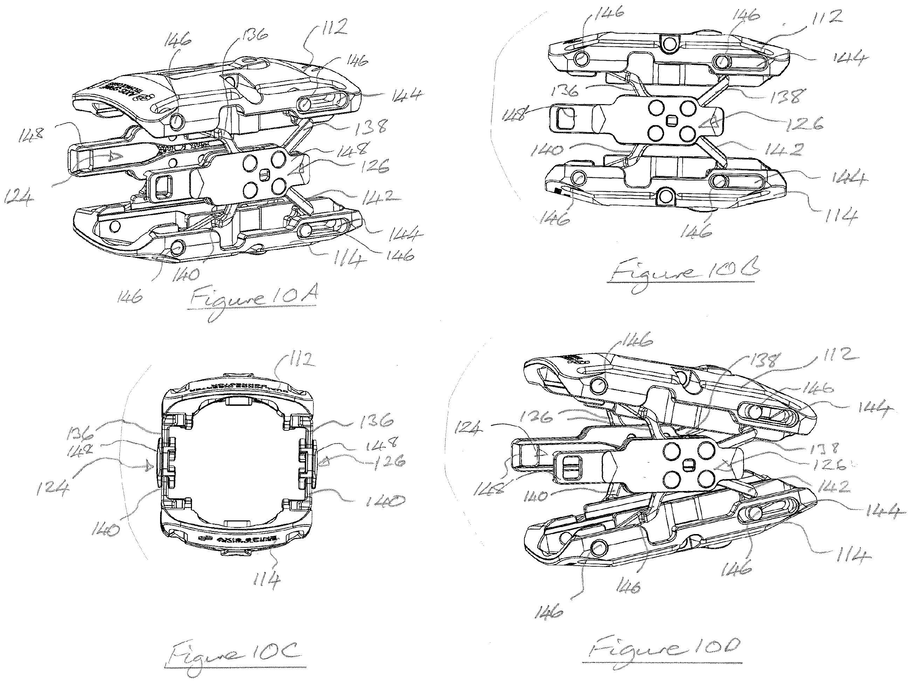

Referring in particular to Figures 10A to 10F, each of the first and second mechanical linkage arrangements 124, 126 comprises a first linkage arm 136, a second linkage arm 138, a third linkage arm 140 and a fourth linkage arm 142. As described further below, distal ends of the first and second linkage arms 136, 138 of the first mechanical linkage arrangement 124 are rotatably coupled to the superior support 112 at spaced apart locations on a first transverse side of the superior support and distal ends of the third and fourth linkage arms 140, 142 of the first mechanical linkage arrangement 124 are rotatably coupled to the inferior support 114 at spaced apart locations on the first transverse side of the inferior support. Distal ends of the first and second linkage arms 136, 138 of the second mechanical linkage arrangement 126 are rotatably coupled to the superior support 112 at spaced apart locations on a second opposite transverse side of the superior support and distal ends of the third and fourth linkage arms 140, 142 of the second mechanical linkage arrangement 126 are rotatably coupled to the inferior support 114 at spaced apart locations on the second transverse side of the inferior support. Furthermore, the rotatable coupling of the distal ends of the second and fourth linkage arms 138, 142 to the superior and inferior supports 112, 114 also allows for relative translation of the superior support and the distal end of the second linkage arm 138 and for relative translation of the inferior support and the distal end of the fourth linkage arm 142. On the other hand, the rotatable coupling of the distal ends of the first and third linkage arms 136, 140 to the superior and inferior supports 112, 114 allows for substantially no relative translation of the superior support and the distal end of the first linkage arm 136 and for substantially no relative translation of the inferior support and the distal end of the third linkage arm 140. This configuration allows for relative translation of the superior support 112 and the distal end of the second linkage arm 138 whereby separation of the distal ends of the first and second linkage arms 136, 138 changes and for relative translation of the inferior support 114 and the distal end of the fourth linkage arm 142 whereby separation of the distal ends of the third and fourth linkage arms 140, 142 changes.

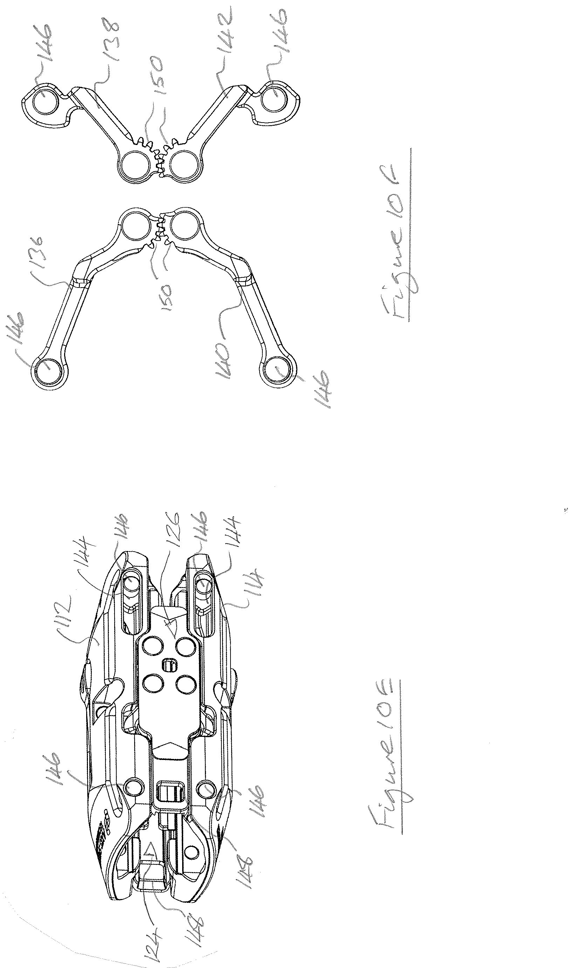

Referring again in particular to Figures 10A, 10B, 10D and 10E, each of the superior support 112 and the inferior support 14 defines a first channel 144 on a first transverse side of the respective support 112, 114 and a second channel 144 on a second opposite transverse side of the respective support. Each channel 144 extends in the direction of the longitudinal axis of the respective one of the superior and inferior arms 116, 118. The distal end of each of the first to fourth linkage arms 136, 138, 140, 142 defines a protrusion 146 which protrudes from the respective distal end in the transverse direction. As the superior and inferior supports 112, 114 move together and apart, the protrusions 146 of the first and third linkage arms 136, 140 rotate in a respective bore defined in the respective support 112, 114 and without relative translation in view of snug reception of each protrusion in the respective bore. Furthermore, the protrusions 146 of the second and fourth linkage arms 138, 142 rotate in and move along their respective channels 144. The first to fourth linkage arms 136, 138, 140, 142 of each of the first and second mechanical linkage arrangements 124, 126 are thus mechanically coupled to one another each at its proximal end whereby a separation is changeable between a) and b), where a) is the distal ends of the first and second linkage arms 136, 138 and b) is the distal ends of the third and fourth linkage arms 140, 142. The change in separation between a) and b) can be appreciated by comparing Figure 10A, which shows the first and second mechanical linkage arrangements 124, 126 when open, with Figure 10E, which shows the first and second mechanical linkage arrangements when closed.

Referring in particular to Figures 10A to 10F, the first to fourth linkage arms 136, 138, 140, 142 are rotatably attached at their proximal ends to a linkage main member 148 such that each of the first to fourth linkage arms rotates about a respective one of first to fourth linkage arm axes and for rotation of the first to fourth linkage arms relative to one another. Each of the first to fourth linkage arm axes is substantially orthogonal to the longitudinal axis of at least one of the superior and inferior arms 116, 118 and to a direction of separation of the superior and inferior arms. The first to fourth linkage arm axes are substantially parallel and spaced apart from one another whereby the first to fourth linkage arm axes extend through a linkage arm plane in which the linkage main member 148 lies and which is parallel to the longitudinal axes of the superior and inferior arms 116, 118 and a direction of separation of the superior and inferior arms. The first to fourth linkage arm axes define in the linkage arm plane and hence in the linkage main member 148 four corners of a rectangle.

Referring to Figure 10F, each of the proximal ends of the first to fourth linkage arms 136, 138, 140, 142 defines a section of a gearwheel 150. Teeth of the gearwheel 150 of the first linkage arm 136 engage with teeth of the gearwheel 150 of the third linkage arm 140 whereby the first and third linkage arms are coupled to rotate together albeit in opposite directions. Teeth of the gearwheel 150 of the second linkage arm 138 engage with teeth of the gearwheel 150 of the fourth linkage arm 142 whereby the second and fourth linkage arms are coupled to rotate together albeit in opposite directions. Configuration of the first and second mechanical linkage arrangements 124,126 in this fashion provides for symmetry of movement of the superior and inferior supports 112, 114 relative to each other, such as during insertion of a core component 130 of an ALIF device between superior and inferior endplates 120, 122 held by the insertion instrument 100. The symmetry of movement is about a plane which extends between the superior and inferior supports 112, 114 and in the transverse direction, and which is equally spaced from the superior and inferior supports. The symmetry of movement of the superior and inferior supports 112, 114 relative to each other corresponds to balancing of forces exerted during insertion of the like of the core component 130 between the superior and inferior supports.

The first and second mechanical linkage arrangements 124, 126 are thus configured to allow relative rotation of the superior and inferior supports 112, 114 about an axis which is substantially orthogonal to the longitudinal axes of the superior and inferior arms and to a direction of separation of the superior and inferior arms 116, 118 further to movement of the superior and inferior supports together and apart but with substantially no relative translation of superior and inferior supports in the direction of the longitudinal axes of the superior and inferior arms. Relative rotation of the superior and inferior supports 112, 114 about the axis which is substantially orthogonal to the longitudinal axes of the superior and inferior arms and to a direction of separation of the superior and inferior arms 116, 118 can be appreciated by comparing Figures 10A and 10D.

The superior and inferior supports 112, 114 and the first and second mechanical linkage arrangements 124, 126 are removably attached to the superior and inferior arms 116, 118. The superior and inferior arms 116, 118 are used with the superior and inferior supports 112, 114 and the first and second mechanical linkage arrangements 124, 126 removed, for example, to align held superior and inferior endplates 120, 122 in the intervertebral space, such as with an alignment tool which is inserted between the superior and inferior arms. The superior and inferior supports 112, 114 and the first and second mechanical linkage arrangements 124,126 are then removably attached to the superior and inferior arms 116, 118, for example, for core component trialling followed by core component insertion.

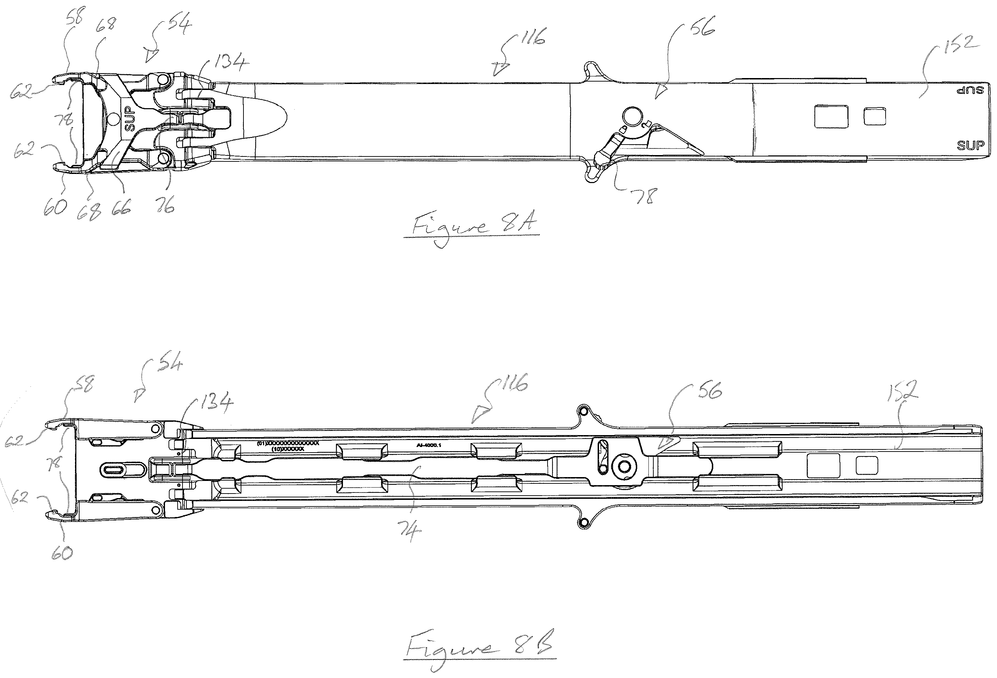

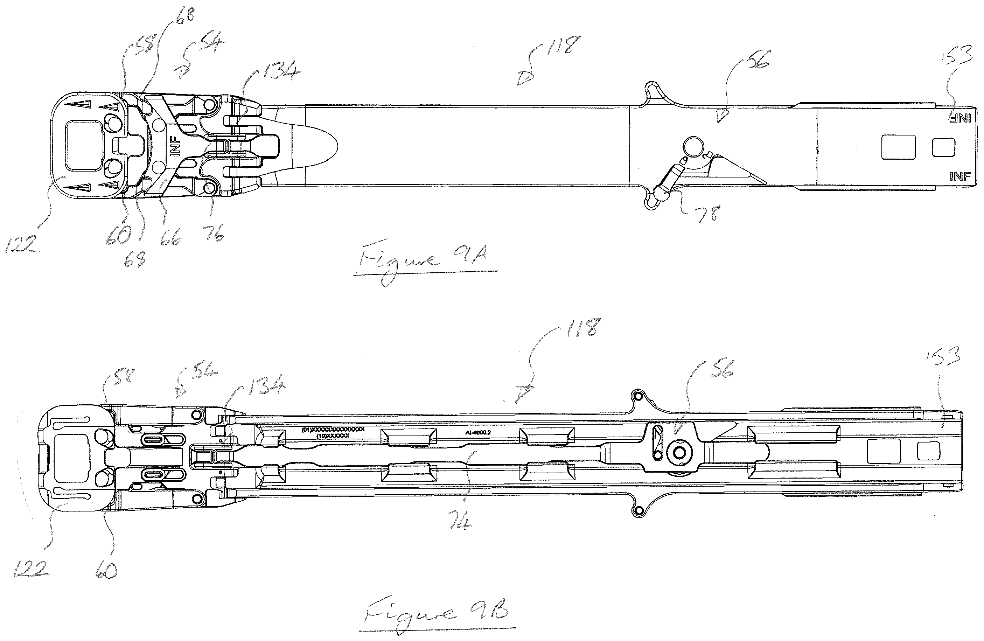

Figure 8A is a view from above of the superior arm 116 of the second embodiment 100 of Figure 7 and when the endplate component gripper is closed and Figure 8B is a view from below of the superior arm of the second embodiment of Figure 7 and when the endplate component gripper is closed. Figure 9A is a view from above of the inferior arm 118 of the second embodiment of Figure 7 and when gripping an inferior component 122 and Figure 9B is a view from below of the inferior arm of the second embodiment of Figure 7 and when gripping the inferior component. As can be appreciated by comparing Figures 8A and 9A, a proximal end 152 of the superior arm 116 is longer than a proximal end 153 of the inferior arm 118. Otherwise, the superior and inferior arms 116, 118 are of the same form and function. Furthermore, the superior and inferior arms 116, 118 of the second embodiment 100 are for the most part of the same form and function as the superior and inferior arms 16, 118 of the first embodiment 10. The reader's attention is directed to the description provided above with reference to Figures 2A to 2C in respect of components common to the first and second embodiments 10, 100. Components of the superior and inferior arms 116, 118 of the second embodiment 100 common with the superior and inferior arms 16, 18 of the first embodiment 10 are designated with common reference numbers in Figures 8A to 9B. Further to this, the distal ends of the superior and inferior arms 116, 118 of the second embodiment 100 are of the same form and function as the distal ends of the superior and inferior arms 16, 18 of the first embodiment 10 in respect of how the distal ends of the two embodiments engage with and grip superior and inferior components 20, 22, 120, 122. The reader's attention is therefore directed to the description provided above with reference to Figures 3A and 3B in respect of the form and function of the distal ends of the superior and inferior arms 116, 118 of the second embodiment 100.

Parts of the insertion instrument 100 are formed from medical grade stainless steel. In other forms of the insertion instrument 100 the superior and inferior arms 116, 118 are formed from a polymeric material, for example PEEK, PAEK, PEAK, PEKK, PE, PU, PPSU, PA, ABS or the like, with or without fibrous material, such as glass, carbon or nylon) to reinforce the polymeric matrix. Forming the superior and inferior arms 116, 118 from a polymeric material improves visibility under x-ray inspection. Further to this, parts of the insertion instrument 100 are coated with diamond like carbon (DLC) to reduce light reflection and thereby improve inspection by way of optical instruments. For the same reason, parts of the insertion instrument 100 are roughened by sandblasting to reduce direct light reflection.

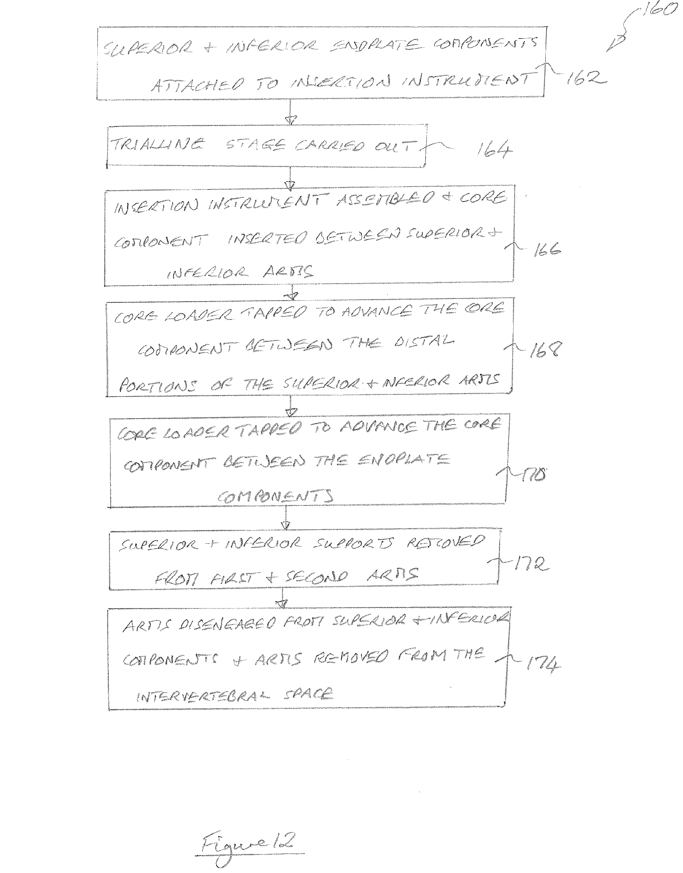

A method of use (not claimed) of the second embodiment 100 of insertion instrument in an ALIF procedure will now be described with reference to Figures 11A to 11E and the flow chart 160 of Figure 12. A superior endplate component 120 is attached to the distal end of the superior arm 116 and an inferior endplate component 122 is attached to the distal end of the inferior arm 118, as described above, 162 whereby the insertion instrument 100 is in the condition shown in Figure 11A. A trialling stage 164 may then be carried out. The trialling stage involves inserting the superior and inferior arms 116, 118 into a surgical aperture and positioning the held superior and inferior endplate components 120, 122 in an intervertebral space. An alignment tool (not shown) may be inserted between the superior and inferior arms 116, 118 whereby a distal end of the alignment tool is used for preliminary alignment of the held superior and inferior endplate components 120, 122 in the intervertebral space to thereby trial the superior and inferior endplate components.

The superior and inferior supports 112, 114 and first and second mechanical linkage arrangements 124, 126 are then attached to the superior and inferior arms 116, 118. As mentioned above, the proximal end 152 of the superior arm 116 is longer than the proximal end 153 of the inferior arm 118. The proximal end 152 of the superior arm 116 defines a keyway at each of its two transverse sides and each of the transverse sides of the core component 130 is shaped to be slidably received in a respective one of the keyways whereby the core component 130 is held by the proximal end 152 and aligned between the superior and inferior arms 116, 118. The keyways extend from the proximal end 152 of the superior arm 116 along the remaining part of the superior arm whereby the core component 130 is held by the superior arm and the core component slides along the superior arm during insertion of the core component. The core loader 128 is then brought into engagement with the thus held core component 130. The core loader 128 with core component 130 attached is pushed forward between the superior and inferior arms until the held core component is just beyond the arm hinges 134, 166 and when the held superior and inferior endplate components 120, 122 are in the intervertebral space whereby the insertion instrument 100 is in the condition shown in Figure 5B. Although not illustrated, a core trialling stage may be carried out after the insertion instrument 100 has been assembled but before insertion of the core component 130.

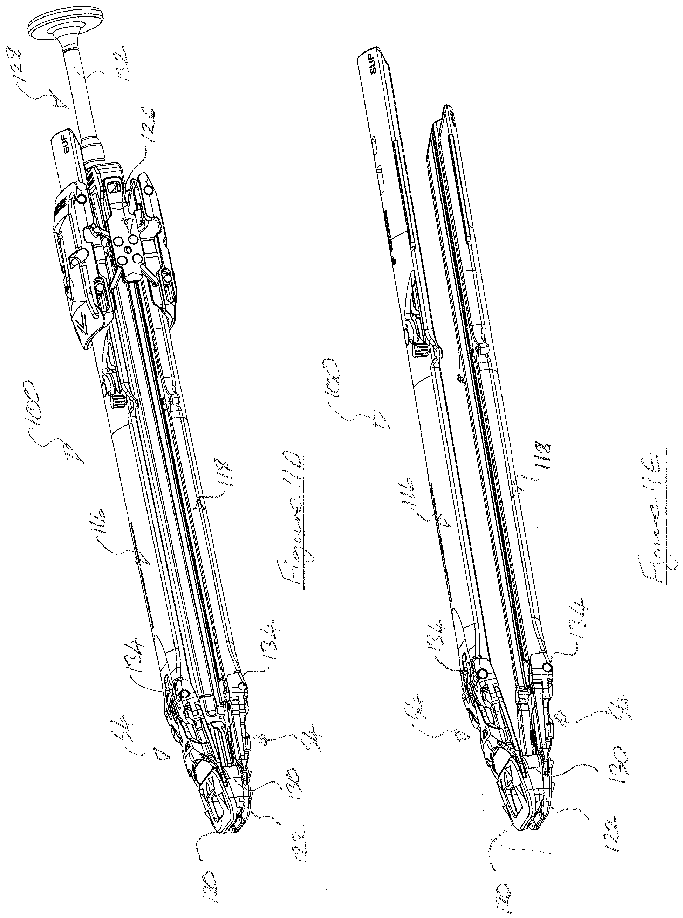

The end of the handle 132 of the core loader 128 is tapped by the clinician to advance the held core component 130 between the distal portions 54 of the superior and inferior arms 168 as shown in Figure 11C. The superior and inferior arms 116, 118 have now been pushed apart to some extent and the distal portions 54 and their held superior and inferior endplate components 120, 122 have now been rotated relative to each other to some extent. The end of the handle 132 of the core loader 128 is tapped further by the clinician to advance the held core component 130 between the held superior and inferior endplate components 120, 122, 170 as shown in Figure 11D. The superior and inferior arms 116, 118 have now been pushed apart to further extent and the distal portions 54 and their held superior and inferior endplate components 120, 122 have now been further rotated to receive the core component 130 between them. The clinician then removes the core loader 128, and the superior and inferior supports 112, 114 and the first and second mechanical linkage arrangements 124, 126 from the superior and inferior arms 116, 118, 172 whereby the insertion instrument 100 is in the condition shown in Figure 11E. Finally, the clinician disengages the distal ends of the superior and inferior arms 116, 118 from the superior and inferior endplate components 120, 122, as described above, and removes the superior and inferior arms from the intervertebral space 174. The assembled ALIF device 82, which is shown in Figure 5E, is thus installed in the intervertebral space.

## Claims

1. An insertion instrument (10, 100) for inserting a modular intervertebral fusion device or components (20, 120) thereof into an intervertebral space, the insertion instrument comprising
a superior support (12, 112) and an inferior support (14, 114) the superior and inferior supports opposing each other;
a superior arm (16, 116) mounted on the superior support;
an inferior arm (18, 118) mounted on the inferior support, the superior and inferior arms extending in generally a same direction from the superior and inferior supports whereby the superior and inferior arms oppose each other, a distal end of each of the superior and inferior arms configured to engage at a respective location on a modular intervertebral fusion device or with a respective component (20, 22, 30, 120, 122, 130) of a modular intervertebral fusion device; and
a mechanical linkage arrangement (24, 26, 124, 126) mechanically coupled to the superior and inferior supports and extending therebetween, the mechanical linkage arrangement comprising first to fourth linkage arms (36, 38, 40, 42, 136, 138, 140, 142) wherein:
each of the superior and inferior arms comprises an arm hinge (34, 134) between its distal end and the respective one of the superior and inferior supports, the arm hinge rotating about an axis which is substantially orthogonal to a longitudinal axis of the respective arm and to a direction of separation of the superior and inferior arms,
distal ends of the first and second linkage arms (36, 38, 136, 138) are rotatably coupled to the superior support at spaced apart locations thereon, the distal end of at least one of the first and second linkage arms coupled to the superior support for relative translation of superior support and respective distal end to thereby change a separation between the distal ends of the first and second linkage arms,
distal ends of the third and fourth linkage arms (40, 42, 140, 142) are rotatably coupled to the inferior support at spaced apart locations thereon, the distal end of at least one of the third and fourth linkage arms coupled to the inferior support for relative translation of inferior support and respective distal end to thereby change a separation between the distal ends of the third and fourth linkage arms, and
the first to fourth linkage arms are mechanically coupled to one another each at a location on the respective linkage arm spaced apart from its distal end whereby a separation is changeable between a) and b), where a) is the distal ends of the first and second linkage arms and b) is the distal ends of the third and fourth linkage arms.

2. The insertion instrument according to claim 1, in which the mechanical linkage arrangement is configured such that the superior and inferior supports move together and apart and such that there is: substantially no relative movement of superior and inferior supports in a direction parallel to the axis of at least one of the arm hinges; and substantially no relative movement of superior and inferior supports in a direction of the longitudinal axis of at least one of the superior and inferior arms.

3. The insertion instrument according to claim 1 or 2, in which the first and second linkage arms are coupled to each other for their relative rotation, and the third and fourth linkage arms are coupled to each other for their relative rotation.

4. The insertion instrument according to claim 3, in which the first and second linkage arms (36, 38) are coupled to each other for their relative rotation about a first linkage arm axis, the third and fourth linkage arms (40, 42) are coupled to each other for their relative rotation about a second linkage arm axis, and the first and second linkage arm axes substantially coincide.

5. The insertion instrument according to claim 4, in which the first and second linkage arm axes are substantially parallel to a longitudinal axis of at least one of the superior and inferior arms.

6. The insertion instrument according to claim 4 or 5, in which the distal ends of the first and third linkage arms are towards one transverse side of the superior and inferior supports (12, 14) and the distal ends of the second and fourth linkage arms are towards the other transverse side of the superior and inferior supports.

7. The insertion instrument according to any one of claims 4 to 6, in which the first and fourth linkage arms are immovably attached to each other whereby the first and fourth linkage arms rotate together relative to the second and third linkage arms, and the second and third linkage arms are immovably attached to each other whereby the second and third linkage arms rotate together relative to the first and fourth linkage arms.

8. The insertion instrument according to claim 7, in which each of the first and fourth linkage arms is immovably attached at their proximal ends to a first axle and such that the first and fourth linkage arms extend from opposite sides of the first axle, and each of the second and third linkage arms is immovably attached at their proximal ends to a second axle and such that the second and third linkage arms extend from opposite sides of the second axle.

9. The insertion instrument according to claim 8, in which the first and second axles are disposed in a direction substantially parallel to the longitudinal axis of at least one of the superior and inferior arms, the first and second axles supported such that they are rotatable relative to each other and about the same axis.

10. The insertion instrument according to any one of claims 4 to 9, in which the distal end of each of the first to fourth linkage arms is coupled to the respective one of the superior and inferior supports for rotation relative to the respective support and for relative translation of respective support and respective distal end, relative translation of respective support and respective distal end being in a direction substantially orthogonal to the longitudinal axis of the respective superior or inferior arm and to the direction of separation of the superior and inferior arms.

11. The insertion instrument according to claim 3, in which the mechanical linkage arrangement (124, 136) is configured to allow relative rotation of the superior and inferior supports (112, 114) about an axis which is substantially orthogonal to the longitudinal axes of the superior and inferior arms (116, 118) and to a direction of separation of the superior and inferior arms.

12. The insertion instrument according to claim 11, in which the first to fourth linkage arms (136, 138, 140, 142) are mechanically coupled to one another for rotation relative to one another, each of the first to fourth linkage arms so coupled at a location on the respective linkage arm spaced apart from its distal end.

13. The insertion instrument according to claim 12, in which the mechanical linkage arrangement is configured for rotation of the first and third linkage arms (136, 140) independently of the second and fourth linkage arms (138, 142), the first and third linkage arms rotating together albeit relative to each other and the second and fourth linkage arms rotating together albeit relative to each other.

14. The insertion instrument according to claim 13, in which the distal end of only one of the first and second linkage arms is coupled to the superior support for relative translation of superior support and the distal end, and the distal end of only one of the third and fourth linkage arms is coupled to the inferior support for relative translation of inferior support and the distal end.

15. The insertion instrument according to claim 13 or 14, in which a proximal end of each of the first to fourth linkage arms defines a gearwheel or part thereof, teeth of the gearwheel of the first linkage arm engaging with teeth of the gearwheel of the third linkage arm, and teeth of the gearwheel of the second linkage arm engaging with teeth of the gearwheel of the fourth linkage arm.

## Patentansprüche

1. Ein Einschubinstrument (10, 100) zum Einschieben einer modularen Zwischenwirbelfusionsvorrichtung oder Komponenten (20, 120) davon in einen Zwischenwirbelraum, wobei das Einschubinstrument Folgendes beinhaltet:
ein oberes Stützelement (12, 112) und ein unteres Stützelement (14, 114), wobei das obere und das untere Stützelement einander gegenüberliegen;
einen oberen Arm (16, 116), der an dem oberen Stützelement angebracht ist;
einen unteren Arm (18, 118), der an dem unteren Stützelement angebracht ist, wobei der obere und der untere Arm sich im Allgemeinen in einer gleichen Richtung von dem oberen und dem unteren Stützelement erstrecken, wodurch der obere und der untere Arm einander gegenüberliegen, wobei ein distales Ende von jedem von dem oberen und dem unteren Arm dazu ausgelegt ist, an einem jeweiligen Ort auf einer modularen Zwischenwirbelfusionsvorrichtung oder mit einer jeweiligen Komponente (20, 22, 30, 120, 122, 130) einer modularen Zwischenwirbelfusionsvorrichtung Eingriff zu nehmen; und
eine mechanische Verknüpfungsanordnung (24, 26, 124, 126), die mechanisch mit dem oberen und dem unteren Stützelement gekoppelt ist und sich zwischen diesen erstreckt, wobei die mechanische Verknüpfungsanordnung einen ersten bis vierten Verknüpfungsarm (36, 38, 40, 42, 136, 138, 140, 142) beinhaltet, wobei:
jeder von dem oberen und dem unteren Arm ein Armscharnier (34, 134) zwischen seinem distalen Ende und dem jeweiligen von dem oberen und dem unteren Stützelement beinhaltet, wobei sich das Armscharnier um eine Achse dreht, die im Wesentlichen senkrecht zu einer Längsachse des jeweiligen Arms und zu einer Richtung der Trennung des oberen und des unteren Arms ist,
die distalen Enden des ersten und zweiten Verknüpfungsarms (36, 38, 136, 138) an beabstandeten Orten davon rotierbar mit dem oberen Stützelement gekoppelt sind, wobei das distale Ende von mindestens einem von dem ersten und zweiten Verknüpfungsarm zur relativen Verschiebung des oberen Stützelements und des jeweiligen distalen Endes mit dem oberen Stützelement gekoppelt ist, um somit eine Trennung zwischen den distalen Enden des ersten und zweiten Verknüpfungsarms zu verändern,
die distalen Enden des dritten und vierten Verknüpfungsarms (40, 42, 140, 142) an beabstandeten Orten davon rotierbar mit dem unteren Stützelement gekoppelt sind, wobei das distale Ende von mindestens einem von dem dritten und vierten Verknüpfungsarm zur relativen Verschiebung des unteren Stützelement und des jeweiligen distalen Endes mit dem unteren Stützelement gekoppelt sind, um somit eine Trennung zwischen den distalen Enden des dritten und vierten Verknüpfungsarms zu verändern, und
der erste bis vierte Verknüpfungsarm jeweils an einem Ort auf dem jeweiligen Verknüpfungsarm mechanisch miteinander gekoppelt sind, der von seinem distalen Ende beabstandet ist, wodurch eine Trennung zwischen a) und b) verändert werden kann, wobei a) die distalen Enden des ersten und zweiten Verknüpfungsarms ist und b) die distalen Enden des dritten und vierten Verknüpfungsarms ist.

2. Einschubinstrument gemäß Anspruch 1, wobei die mechanische Verknüpfungsanordnung so ausgelegt ist, dass sich das obere und das untere Stützelement zusammen und auseinander bewegen, und so, dass: es im Wesentlichen keine Bewegung des oberen und des unteren Stützelements in einer Richtung gibt, die parallel zu der Achse von mindestens einem der Armscharniere ist; und es im Wesentlichen keine relative Bewegung des oberen und des unteren Stützelements in einer Richtung der Längsachse von mindestens einem des oberen und des unteren Arms gibt.

3. Einschubinstrument gemäß Anspruch 1 oder 2, wobei der erste und der zweite Verknüpfungsarm für ihre relative Rotation miteinander gekoppelt sind und der dritte und der vierte Verknüpfungsarm für ihre relative Rotation miteinander gekoppelt sind.

4. Einschubinstrument gemäß Anspruch 3, wobei der erste und der zweite Verknüpfungsarm (36, 38) für ihre relative Rotation um eine erste Verknüpfungsarmachse miteinander gekoppelt sind, der dritte und der vierte Verknüpfungsarm (40, 42) für ihre relative Rotation um eine zweite Verknüpfungsarmachse miteinander gekoppelt sind und die erste und die zweite Verknüpfungsarmachse im Wesentlichen zusammenfallen.

5. Einschubinstrument gemäß Anspruch 4, wobei die erste und zweite Verknüpfungsarmachse im Wesentlichen parallel zu einer Längsachse von mindestens einem von dem oberen und dem unteren Arm sind.

6. Einschubinstrument gemäß Anspruch 4 oder 5, wobei die distalen Enden des ersten und des dritten Verknüpfungsarms hin zu einer Querseite des oberen und des unteren Stützelements (12, 14) liegen und die distalen Enden des zweiten und des vierten Verknüpfungsarms hin zu der anderen Querseite des oberen und des unteren Stützelements liegen.

7. Einschubinstrument gemäß einem der Ansprüche 4 bis 6, wobei der erste und der vierte Verknüpfungsarm unbeweglich aneinander befestigt sind, wodurch sich der erste und der vierte Verknüpfungsarm relativ zu dem zweiten und dem dritten Verknüpfungsarm zusammen drehen, und der zweite und der dritte Verknüpfungsarm unbeweglich aneinander befestigt sind, wodurch sich der zweite und der dritte Verknüpfungsarm relativ zu dem ersten und dem vierten Verknüpfungsarm relativ zusammen drehen.

8. Einschubinstrument gemäß Anspruch 7, wobei jeder von dem ersten und vierten Verknüpfungsarm an ihren proximalen Enden unbeweglich an einer ersten Achswelle befestigt ist und zwar so, dass sich der erste und der vierte Verknüpfungsarm von entgegengesetzten Seiten der ersten Achswelle erstrecken, und jeder von dem zweiten und dem dritten Verknüpfungsarm an ihren proximalen Enden unbeweglich an einer zweiten Achswelle befestigt ist, und zwar so, dass sich der zweite und der dritte Verknüpfungsarm von entgegengesetzten Seiten der zweiten Achswelle erstrecken.

9. Einschubinstrument gemäß Anspruch 8, wobei die erste und die zweite Achswelle in einer Richtung angeordnet sind, die im Wesentlichen parallel zu der Längsachse von mindestens einem von dem oberen und dem unteren Arm ist, wobei die erste und die zweite Achswelle so gestützt sind, dass sie relativ zueinander und um die gleiche Achse gedreht werden können.

10. Einschubinstrument gemäß einem der Ansprüche 4 bis 9, wobei das distale Ende von jedem von dem ersten bis vierten Verknüpfungsarm mit dem jeweiligen von dem oberen und dem unteren Stützelement für eine Rotation relativ zu dem jeweiligen Stützelement und für eine relative Verschiebung des jeweiligen Stützelements und jeweiligen distalen Endes gekoppelt ist, wobei die relative Verschiebung des jeweiligen Stützelements und jeweiligen distalen Endes in einer Richtung ist, die im Wesentlichen senkrecht zu der Längsachse des jeweiligen oberen oder unteren Arms und zu der Richtung der Trennung des oberen und des unteren Arms ist.

11. Einschubinstrument gemäß Anspruch 3, wobei die mechanische Verknüpfungsanordnung (124, 136) dazu ausgelegt ist, eine relative Rotation des oberen und des unteren Stützelements (112, 114) um eine Achse zuzulassen, die im Wesentlichen senkrecht zu den Längsachsen des oberen und des unteren Arms (116, 118) und zu einer Richtung der Trennung des oberen und des unteren Arms ist.

12. Einschubinstrument gemäß Anspruch 11, wobei der erste bis vierte Verknüpfungsarm (136, 138, 140, 142) für eine Rotation relativ zueinander mechanisch miteinander gekoppelt sind, wobei jeder von dem ersten bis vierten Verknüpfungsarm an einem Ort auf dem jeweiligen Verknüpfungsarm, der von seinem distalen Ende beabstandet ist, so gekoppelt ist.

13. Einschubinstrument gemäß Anspruch 12, wobei die mechanische Verknüpfungsanordnung zur Rotation des ersten und des dritten Verknüpfungsarms (136, 140) unabhängig von dem zweiten und dem vierten Verknüpfungsarm (138, 142) ausgelegt ist, wobei der erste und der dritte Verknüpfungsarm sich zusammen, wenngleich relativ zueinander drehen und der zweite und der vierte Verknüpfungsarm sich zusammen, wenngleich relativ zueinander drehen.

14. Einschubinstrument gemäß Anspruch 13, wobei das distale Ende von nur einem von dem ersten und dem zweiten Verknüpfungsarm für eine relative Verschiebung des oberen Stützelements und des distalen Endes mit dem oberen Stützelement gekoppelt ist und das distale Ende von nur einem von dem dritten und dem vierten Verknüpfungsarm für eine relative Verschiebung des unteren Stützelements und des distalen Endes mit dem unteren Stützelement gekoppelt ist.

15. Einschubinstrument gemäß Anspruch 13 oder 14, wobei ein proximales Ende von jedem von dem ersten bis vierten Verknüpfungsarm ein Zahnrad oder einen Teil davon definiert, wobei Zähne des Zahnrads des ersten Verknüpfungsarms Eingriff mit Zähnen des Zahnrads des dritten Verknüpfungsarms nehmen und Zähne des Zahnrads des zweiten Verknüpfungsarms Eingriff mit Zähnen des Zahnrads des vierten Verknüpfungsarms nehmen.

## Revendications

1. Instrument d'insertion (10, 100) pour l'insertion d'un dispositif de spondylodèse modulaire ou de composants (20, 120) de celui-ci dans un espace intersomatique, l'instrument d'insertion comprenant :
un support supérieur (12, 112) et un support inférieur (14, 114), les supports supérieur et inférieur étant placés en regard l'un de l'autre ;
un bras supérieur (16, 116) adapté sur le support supérieur ;
un bras inférieur (18, 118) adapté sur le support inférieur, les bras supérieur et inférieur s'étendant globalement dans une même direction à partir des supports supérieur et inférieur, les bras supérieur et inférieur étant ainsi placés en regard l'un de l'autre, une extrémité distale de chacun des bras supérieur et inférieur étant conçue pour se mettre en prise au niveau d'un emplacement respectif sur un dispositif de spondylodèse modulaire ou avec un composant respectif (20, 22, 30, 120, 122, 130) d'un dispositif de spondylodèse modulaire ; et
un système de liaison mécanique (24, 26, 124, 126) accouplé mécaniquement aux supports supérieur et inférieur et s'étendant entre ceux-ci, le système de liaison mécanique comprenant des premier à quatrième bras de liaison (36, 38, 40, 42, 136, 138, 140, 142), dans lequel :
chacun des bras supérieur et inférieur comprend une charnière de bras (34, 134) entre son extrémité distale et le support respectif parmi les supports supérieur et inférieur, la charnière de bras tournant autour d'un axe qui est sensiblement orthogonal à un axe longitudinal du bras respectif et à une direction de séparation des bras supérieur et inférieur,
des extrémités distales des premier et deuxième bras de liaison (36, 38, 136, 138) sont accouplées à rotation au support supérieur à des emplacements espacés sur celui-ci, l'extrémité distale d'au moins un des premier et deuxième bras de liaison étant accouplée au support supérieur pour permettre une translation relative du support supérieur et de l'extrémité distale respective de façon à modifier ainsi une séparation entre les extrémités distales des premier et deuxième bras de liaison,
des extrémités distales des troisième et quatrième bras de liaison (40, 42, 140, 142) sont accouplées à rotation au support inférieur à des emplacements espacés sur celui-ci, l'extrémité distale d'au moins un des troisième et quatrième bras de liaison étant accouplée au support inférieur pour permettre une translation relative du support inférieur et de l'extrémité distale respective de façon à modifier ainsi une séparation entre les extrémités distales des troisième et quatrième bras de liaison, et
les premier à quatrième bras de liaison sont accouplés mécaniquement les uns aux autres chacun à un emplacement sur le bras de liaison respectif espacé de son extrémité distale, de façon qu'une séparation peut ainsi être modifiée entre a) et b), à savoir a) les extrémités distales des premier et deuxième bras de liaison et b) les extrémités distales des troisième et quatrième bras de liaison.

2. Instrument d'insertion selon la revendication 1, dans lequel le système de liaison mécanique est conçu de telle sorte que les supports supérieur et inférieur se rapprochent et s'éloignent l'un de l'autre et de telle sorte qu'il n'y a : sensiblement pas de mouvement relatif des supports supérieur et inférieur dans une direction parallèle à l'axe d'au moins une des charnières de bras ; et sensiblement pas de mouvement relatif des supports supérieur et inférieur dans une direction de l'axe longitudinal d'au moins un des bras supérieur et inférieur.

3. Instrument d'insertion selon la revendication 1 ou 2, dans lequel les premier et deuxième bras de liaison sont accouplés l'un à l'autre pour permettre une rotation relative de ceux-ci, et les troisième et quatrième bras de liaison sont accouplés l'un à l'autre pour permettre une rotation relative de ceux-ci.

4. Instrument d'insertion selon la revendication 3, dans lequel les premier et deuxième bras de liaison (36, 38) sont accouplés l'un à l'autre pour permettre une rotation relative de ceux-ci autour d'un premier axe de bras de liaison, les troisième et quatrième bras de liaison (40, 42) sont accouplés l'un à l'autre pour permettre une rotation relative de ceux-ci autour d'un second axe de bras de liaison, et les premier et second axes de bras de liaison coïncident sensiblement.

5. Instrument d'insertion selon la revendication 4, dans lequel les premier et second axes de bras de liaison sont sensiblement parallèles à un axe longitudinal d'au moins un des bras supérieur et inférieur.

6. Instrument d'insertion selon la revendication 4 ou 5, dans lequel les extrémités distales des premier et troisième bras de liaison se trouvent vers un côté transversal des supports supérieur et inférieur (12, 14) et les extrémités distales des deuxième et quatrième bras de liaison se trouvent vers l'autre côté transversal des supports supérieur et inférieur.

7. Instrument d'insertion selon l'une quelconque des revendications 4 à 6, dans lequel les premier et quatrième bras de liaison sont attachés de manière fixe l'un à l'autre, de façon que les premier et quatrième bras de liaison tournent ainsi conjointement relativement aux deuxième et troisième bras de liaison, et les deuxième et troisième bras de liaison sont attachés de manière fixe l'un à l'autre, de façon que les deuxième et troisième bras de liaison tournent ainsi conjointement relativement aux premier et quatrième bras de liaison.

8. Instrument d'insertion selon la revendication 7, dans lequel chacun des premier et quatrième bras de liaison est attaché de manière fixe, au niveau de leurs extrémités proximales, à une première broche et de telle sorte que les premier et quatrième bras de liaison s'étendent à partir de côtés opposés de la première broche, et chacun des deuxième et troisième bras de liaison est attaché de manière fixe, au niveau de leurs extrémités proximales, à une seconde broche et de telle sorte que les deuxième et troisième bras de liaison s'étendent à partir de côtés opposés de la seconde broche.

9. Instrument d'insertion selon la revendication 8, dans lequel les première et seconde broches sont disposées dans une direction sensiblement parallèle à l'axe longitudinal d'au moins un des bras supérieur et inférieur, les première et seconde broches étant soutenues de telle sorte qu'elles soient rotatives l'une par rapport à l'autre et autour du même axe.

10. Instrument d'insertion selon l'une quelconque des revendications 4 à 9, dans lequel l'extrémité distale de chacun des premier à quatrième bras de liaison est accouplée au support respectif parmi les supports supérieur et inférieur pour permettre une rotation relativement au support respectif et pour permettre une translation relative du support respectif et de l'extrémité distale respective, la translation relative du support respectif et de l'extrémité distale respective se faisant dans une direction sensiblement orthogonale à l'axe longitudinal du bras supérieur ou inférieur respectif et à la direction de séparation des bras supérieur et inférieur.

11. Instrument d'insertion selon la revendication 3, dans lequel le système de liaison mécanique (124, 136) est conçu pour permettre une rotation relative des supports supérieur et inférieur (112, 114) autour d'un axe qui est sensiblement orthogonal aux axes longitudinaux des bras supérieur et inférieur (116, 118) et à une direction de séparation des bras supérieur et inférieur.

12. Instrument d'insertion selon la revendication 11, dans lequel les premier à quatrième bras de liaison (136, 138, 140, 142) sont accouplés mécaniquement les uns aux autres pour permettre une rotation relative des uns par rapport aux autres, chacun des premier à quatrième bras de liaison étant accouplé de cette manière au niveau d'un emplacement sur le bras de liaison respectif espacé de son extrémité distale.

13. Instrument d'insertion selon la revendication 12, dans lequel le système de liaison mécanique est conçu pour permettre une rotation des premier et troisième bras de liaison (136, 140) indépendamment des deuxième et quatrième bras de liaison (138, 142), les premier et troisième bras de liaison tournant conjointement bien que l'un par rapport à l'autre et les deuxième et quatrième bras de liaison tournant conjointement bien que l'un par rapport à l'autre.

14. Instrument d'insertion selon la revendication 13, dans lequel l'extrémité distale d'un seul des premier et deuxième bras de liaison est accouplée au support supérieur pour permettre une translation relative du support supérieur et de l'extrémité distale, et l'extrémité distale d'un seul des troisième et quatrième bras de liaison est accouplée au support inférieur pour permettre une translation relative du support inférieur et de l'extrémité distale.

15. Instrument d'insertion selon la revendication 13 ou 14, dans lequel une extrémité proximale de chacun des premier à quatrième bras de liaison définit une roue dentée ou une partie de celle-ci, les dents de la roue dentée du premier bras de liaison s'engrenant avec les dents de la roue dentée du troisième bras de liaison, et les dents de la roue dentée du deuxième bras de liaison s'engrenant avec les dents de la roue dentée du quatrième bras de liaison.
